(19) Europäisches Patentamt
European Patent Office
Office européen des brevets

(11) Publication number: **0 249 223 B1**

(12) # EUROPEAN PATENT SPECIFICATION

(45) Date of publication of patent specification: **14.10.92**

(51) Int. Cl.5: **C07K 5/06**, A61K 37/64

(21) Application number: **87108410.9**

(22) Date of filing: **11.06.87**

(54) **Novel antihypertensive agent.**

(30) Priority: **13.06.86 US 873754**

(43) Date of publication of application:
**16.12.87 Bulletin 87/51**

(45) Publication of the grant of the patent:
**14.10.92 Bulletin 92/42**

(84) Designated Contracting States:
**AT BE CH DE ES FR GB GR IT LI LU NL SE**

(56) References cited:
**EP-A- 0 107 095**
**EP-A- 0 166 357**

(73) Proprietor: **MERRELL DOW PHARMACEUTICALS INC.**
**2110 East Galbraith Road**
**Cincinnati Ohio 45215-6300(US)**

(72) Inventor: **Flynn, Gary A.**
**7121 Euclid Drive**
**Cincinnati Ohio 45243(US)**
Inventor: **Beight, Douglas W.**
**645 Glensprings Drive**
**Cincinnati Ohio 45246(US)**

(74) Representative: **Sgarbi, Renato et al**
**GRUPPO LEPETIT S.p.A. Patent and Trademark Department Via Roberto Lepetit, 34**
**I-21040 Gerenzano (Varese)(IT)**

EP 0 249 223 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to derivatives of fused tricyclic lactams, to the intermediates and processes useful for their preparation and to pharmaceutical compositions for inhibiting the angiotensin converting enzyme and the treatment of hypertension.

EP-A-0 107 095 and EP-A-0 166 357 disclose benzofused bicyclic lactams which are useful as antihypertensive agents and as cholecystokinin antagonists.

In particular this invention relates to fused tricyclic lactams of the formula

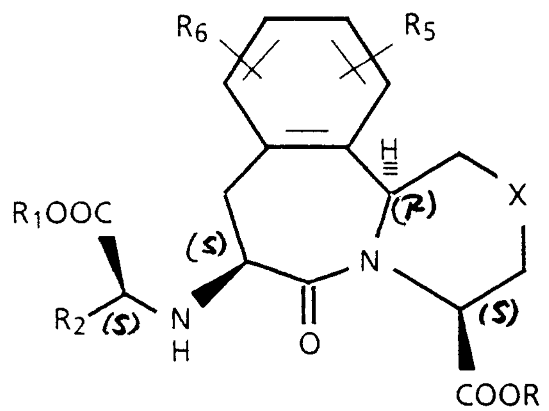

(I)

and the pharmaceutically acceptable salts thereof wherein

R and $R_1$ are independently

(a) hydrogen;

(b) $C_1$-$C_6$ alkyl;

(c) substituted $C_1$-$C_6$ alkyl wherein the substituents are hydroxy, $C_1$-$C_4$ alkyloxy and di-($C_1$-$C_4$)-alkylamino;

(d) $C_6$ to $C_{12}$ aryl;

(e) substituted $C_6$ to $C_{12}$ aryl wherein the substituents are $C_1$-$C_6$ alkyl, halo (F, Cl, Br, I)and $C_1$-$C_4$ alkyloxy;

(f) hetero ($C_4$ to $C_9$)aryl wherein the heteroatom can be one of O, N or S;

(g) substituted hetero ($C_4$ to $C_9$)aryl wherein the heteroatom can be one of O, N or S and the substituents are $C_1$-$C_6$ alkyl, halo (F, $C_1$, Br, I) and $C_1$-$C_4$ alkyloxy;

$R_2$ is

(a) hydrogen;

(b) $C_1$-$C_8$ straight or branched alkyl;

(c) $C_2$-$C_8$ straight or branched alkenyl;

(d) $C_2$-$C_8$ straight or branched alkynyl;

(e) $C_3$-$C_{10}$ cycloalkyl;

(f) $C_6$ or $C_{10}$ aryl ($C_1$-$C_4$)alkyl;

(g) substituted $C_1$-$C_8$ alkyl which contains a $CONR_2$, $SO_2NR_2$, $NRCONR_2$, $OCONR_2$, NRCOOR or $NR_2$ group, wherein R is as defined above;

$R_5$ and $R_6$ each independently are

(a) hydrogen, hydroxy;

(b) halo (F, Br, Cl, I);

(c) $C_1$-$C_6$ alkyl;

(d) $C_1$-$C_6$ alkyloxy; and X is $CH_2$, O or S.

The alkyl groups are represented by such groups as, for example methyl, ethyl, isobutyl. The cycloalkyl groups include, for example, cyclobutyl, cyclopentyl, cyclohexyl. The $C_6$ to $C_{12}$ aryl groups include phenyl, naphthyl, indenyl, biphenyl and benzofused cycloalkyl groups such as, for example, indanyl and 1,2,3,4,-tetrahydronaphthyl. The $C_4$ to $C_9$ heteroaryl groups include such compounds as, for example, pyridyl, thienyl, furyl, imidazolyl and thiazolyl as well as any bicyclic group in which any of the above heterocyclic rings is fused to another aromatic or heterocyclic ring such as, for example, indolyl, quinolinyl, isoquinolinyl,

benzimidazolyl, 1,5-naphthyridyl and quinoxalinyl.

Preferred compounds are those compounds wherein R is hydrogen, $R_1$ is hydrogen or preferably an alkyl radical, preferably ethyl, $R_2$ is $C_6$ aryl-$C_1$-$C_4$ alkyl, most preferably phenethyl, $R_5$ and $R_6$ are both hydrogen, $C_{1-6}$ alkoxy or represent monohydroxy or dihydroxy substituents on the benzenoid moiety.

The compounds of this invention may be prepared by a series of reaction steps which, per se, are analogously known in the art. In general it is preferred to initiate the sequence utilizing an appropriate N-protected arylalanine which, when converted to its acid chloride, is coupled with an appropriate amine according to the well-known principles of the Schotten-Baumann reaction. Before the nitrogen atom attached to the 3-position of the benzazepin-2-one moiety is subjected to coupling procedures, the intermediates are subjected to a Friedel-Crafts cyclization reaction. Following the cyclization, the N-protecting group, (e.g., the phthalimido moiety) is removed and the nitrogen atom is subjected to standard coupling procedures to produce the desired $R_1$, $R_2$ bearing compounds of formula I.

The acid chloride derivatives of the N-protected arylalanine (III) are coupled by treatment with an aminovinyl chloride (IXa) or an -OH protected vinyl amino acid (IXb) according to the Schotten-Baumann reaction to produce intermediates (Xa and Xb respectively). These intermediates are treated with ozone in methylene chloride containing an alcohol at -78°C, quenched with dimethylsulfide and pyridine, and the isolated products are treated with trifluoroacetic acid/methylene chloride at reflux temperatures to produce acylenamines (XIa) and (XIb) which are then treated with the $CF_3SO_3H$ according to the Friedel-Crafts cyclization procedure to produce compounds XIIa and XIIb. In effecting the Friedel-Crafts cyclization it is preferred to utilize a Lewis acid preferably chosen from perfluoroalkyl sulfonic acids, such as for example trifluoromethane sulfonic acid, pentafluoroethane sulfonic acid and heptafluoropropane sulfonic acid. These reactions are shown in Reaction Scheme A.

Reaction Scheme A

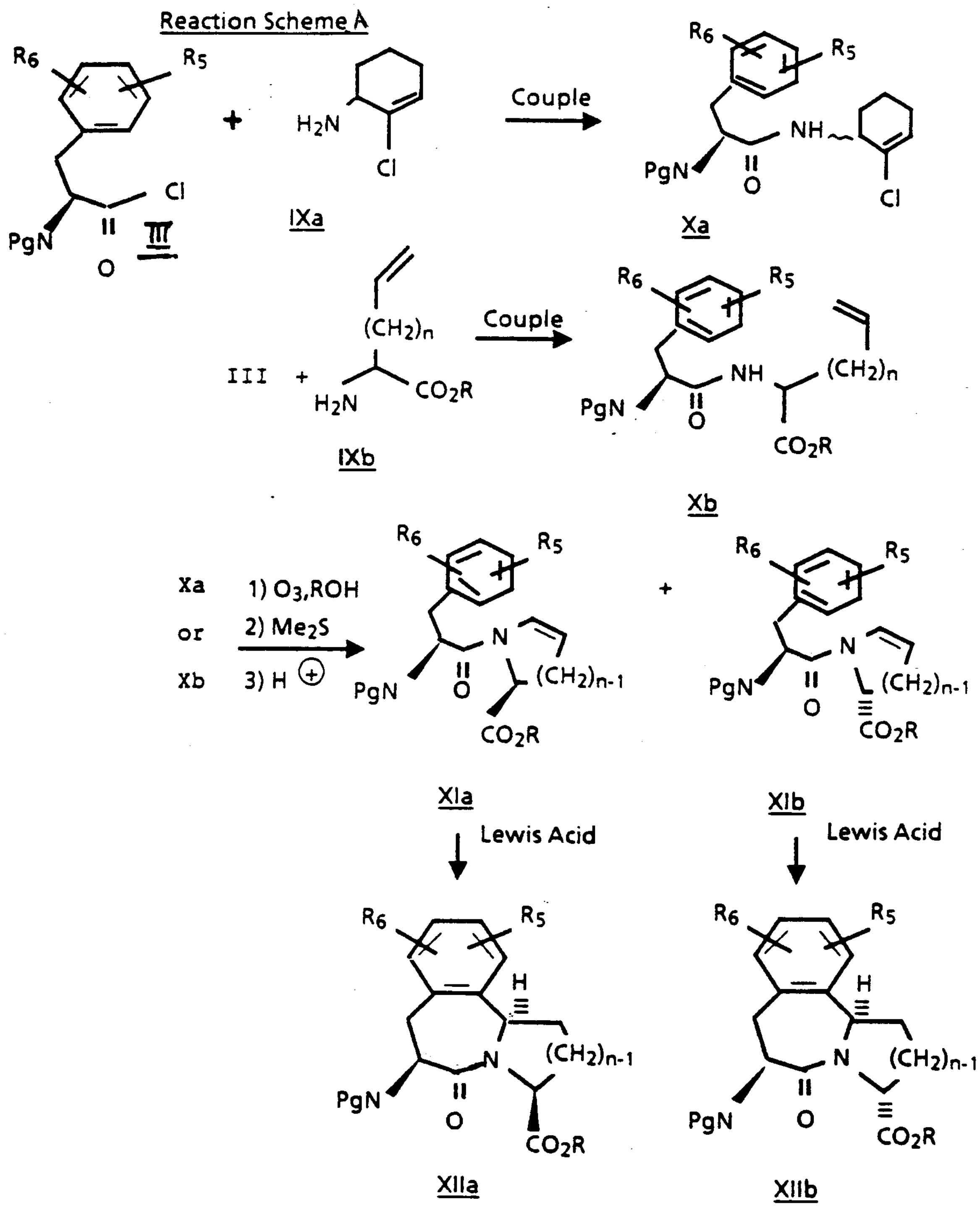


wherein R, $R_5$, $R_6$, are as defined for formula (I), Pg is a N-protecting group (preferably forming a phthalimido), n is 3.

In those instances wherein it is desired to produce compounds wherein the fused ring bears a sulfur or oxygen in that ring, then such compounds are prepared according to Reaction Scheme B and C.

Reaction Scheme B

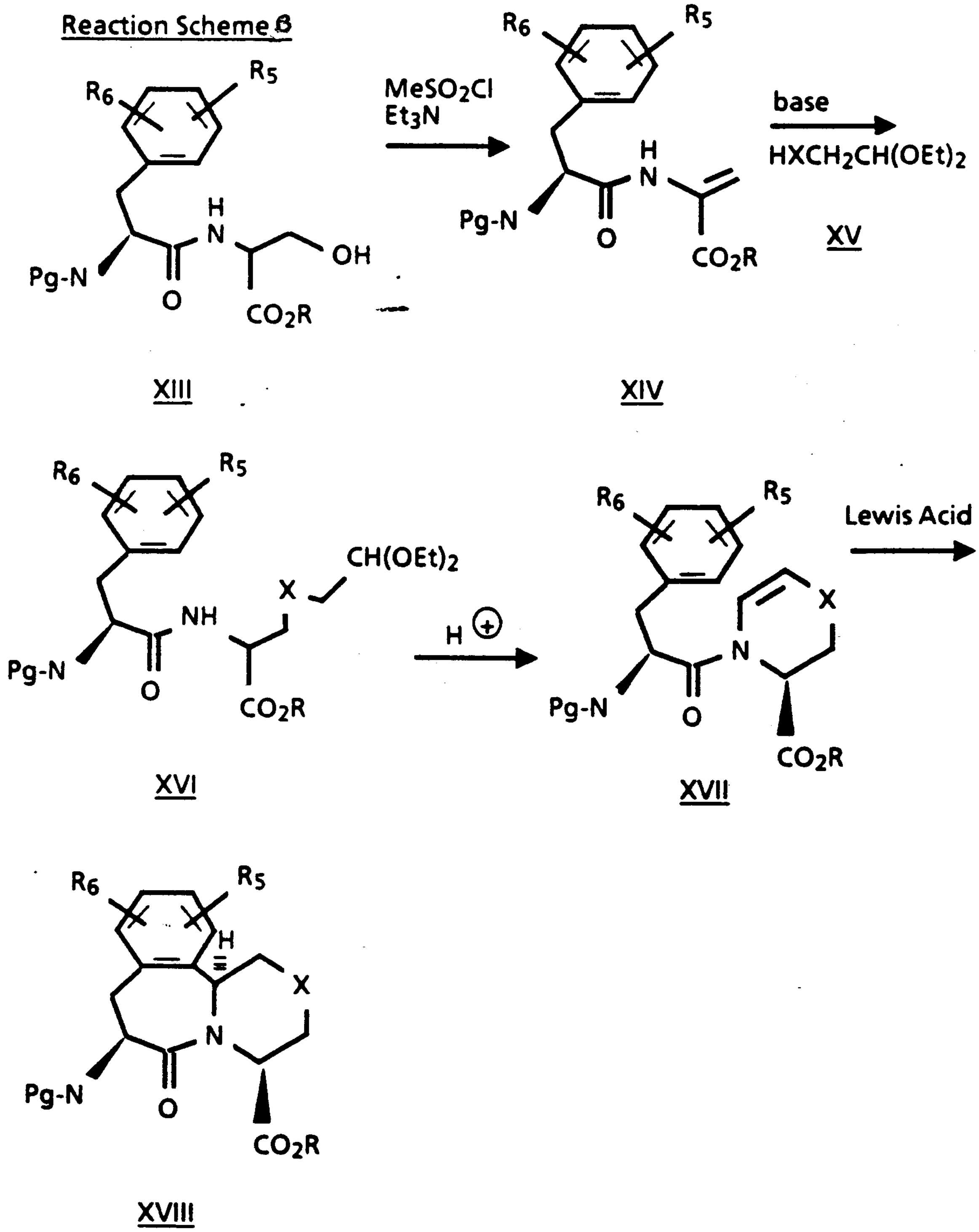


XIII XIV XV XVI XVII XVIII

wherein Pg is a N-protecting group, preferably a phthalimido, R, $R_5$, $R_6$ are as defined for formula (I) and X is S or O.

In the foregoing Reaction Scheme B the N-protected phenylalanyl serine ester (XIII) is converted (in situ), to its mesylate and then eliminated to give the dehydroalanine (XIV) by treatment with methane sulfonyl chloride in triethylamine in an inert solvent, e.g., dichloromethane. Conjugate addition of $HXCH_2CH(OEt)_2$ (XV) with the dehydroalanine intermediates (XIV) gives a Michael adduct (XVI) which is cyclized to acylenamines (XVII) by the action of trifluoroacetic acid in dichloromethane. Final cyclization to (XVIII) is effected by the above described Friedel-Crafts reaction.

Alternately in the specific instance wherein X is sulfur then intermediates XVIa may be prepared by alkylation of L-cysteine ethyl ester (XIX) with bromoacetaldehyde diethyl acetal and sodium iodide, in DMF, and catalyzed with a suitable base such as triethylamine or sodium hydride. The resulting free amine (XX)

is coupled to an N-protected (preferably phthaloyl) L-phenylalanine (XXI) by action of standard coupling reagents such as N-carbethoxy-2-ethoxy-1,2-dihydroquinoline (EEDQ) to give intermediates (XVIa) which are cyclized according to standard Lewis acid-Friedel-Crafts cyclization procedures.

Reaction Scheme C

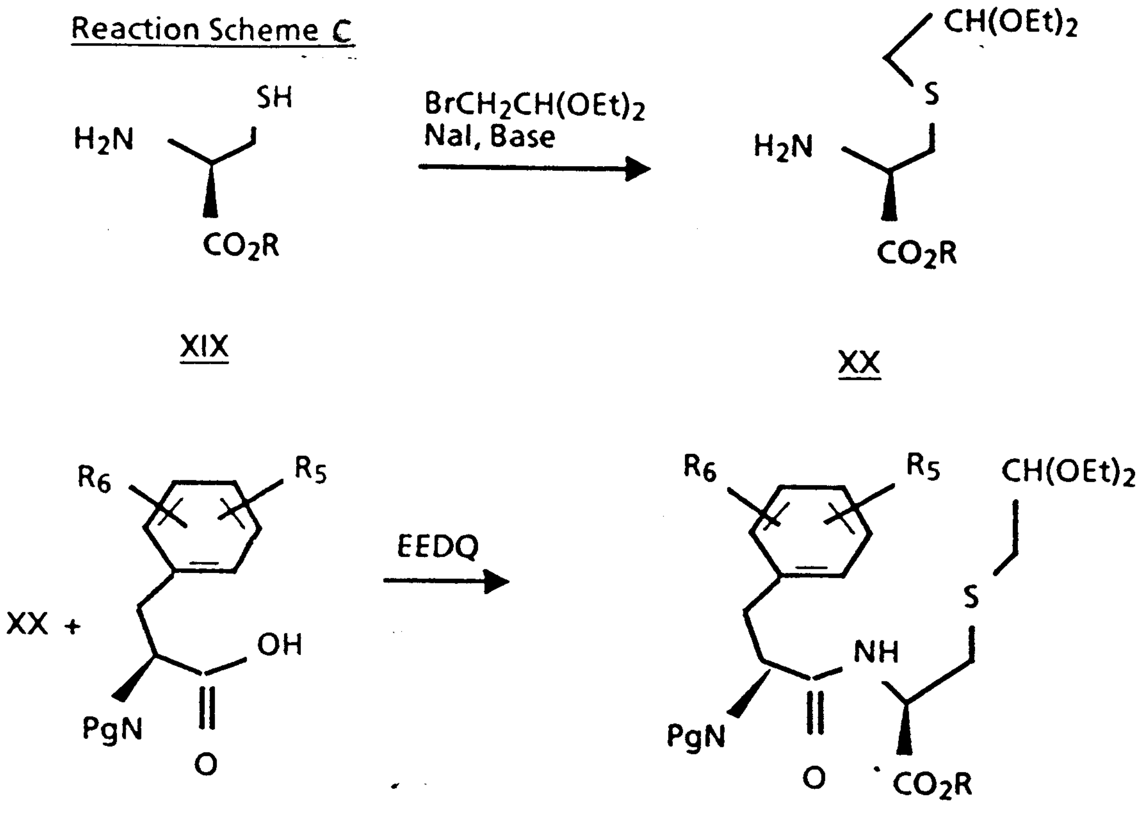


XIX

XX

XXI

XVIa

wherein R, $R_5$, $R_6$ and Pg are as previously defined.

In those reactions of Reaction Schemes A, B and C wherein a fused lactam is prepared by a Friedel-Crafts cyclization procedure it is, of course, preferred to utilize a perfluoroalkyl sulfonic acid.

Once the fused lactams (e.g., XIIa, XIIb and XVIII) have been prepared, the N-protecting group is removed so that the appropriate side chain may be coupled to the free amine. This deprotection may be effected by standard and well known procedures. In the instance wherein the protecting group is phthaloyl, it is convenient to remove the phthalimido moiety by reaction with hydrazine hydrate by techniques well-known in the art. The deprotected fused lactams are as depicted by the following structure

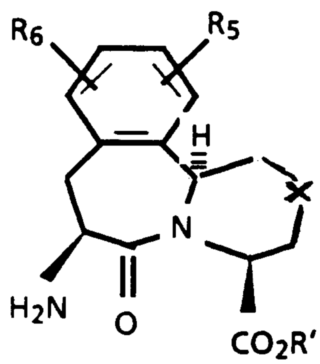

XXII

wherein R′ is as defined for R except that it is other than H.

Although any of the known procedures generally used for such couplings may be utilized, the preferred methods for such couplings are shown in Reaction Scheme D.

Reaction Scheme D

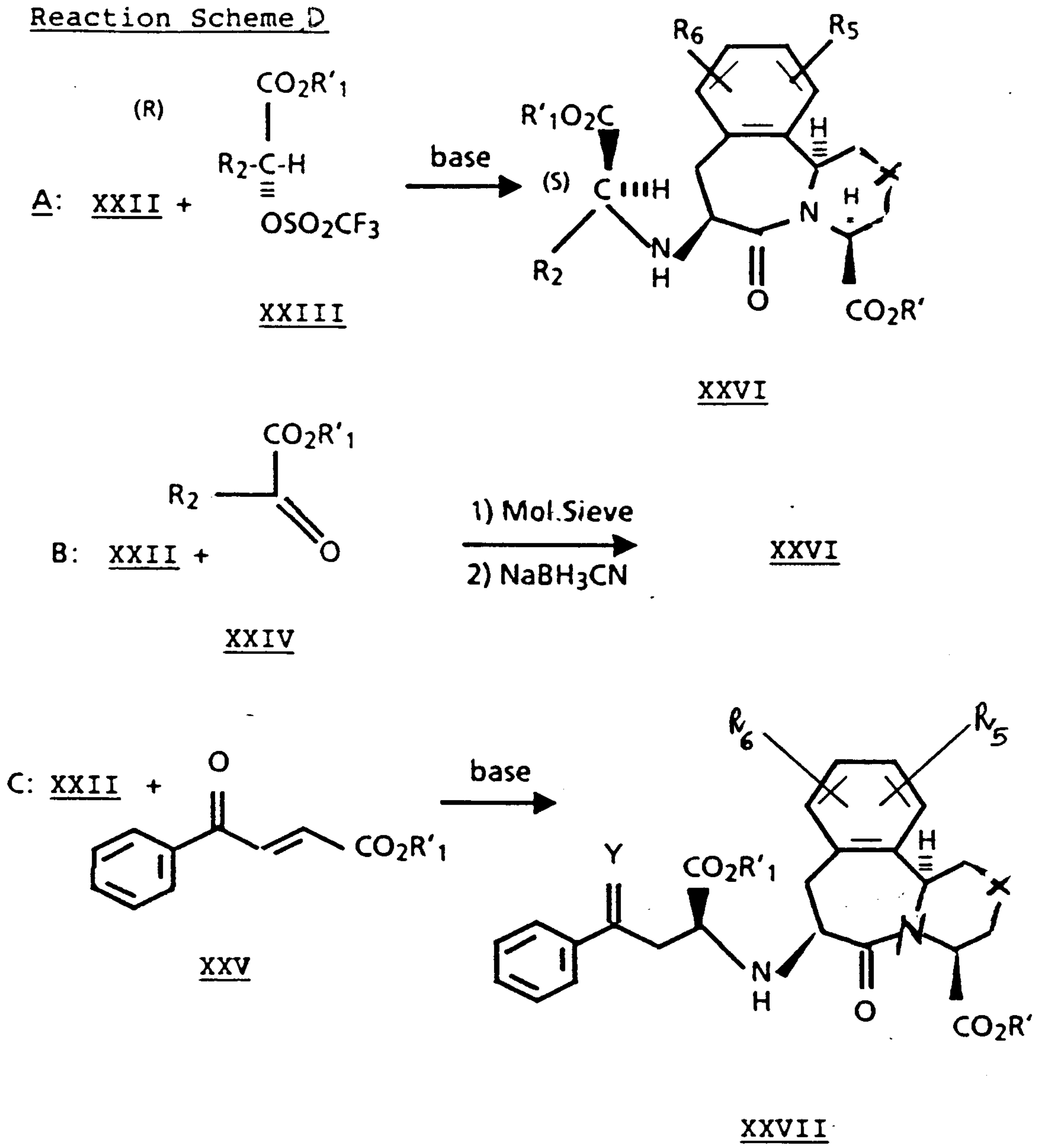


A: XXII + XXIII → XXVI

B: XXII + XXIV → XXVI

C: XXII + XXV → XXVII

wherein R' and $R'_1$ are as previously defined except they cannot be H, and $R_2$, $R_5$, $R_6$ and X are as previously defined.

Method A of Scheme D entails the displacement reaction with a (R) triflate (XXIII) wherein the fused lactams are contacted with the triflate in the presence of a base, e.g., triethylamine, but more preferably in the presence of a "proton sponge", i.e., 1,8-bis-(dimethylamino)-naphthalene, to produce compounds XXVI.

Method B entails the use of a keto ester (XXIV) in ethanol (or other alcoholic solvent) with a molecular sieve to form a Schiff's base which is reduced to compounds XXVI, preferably using sodium cyanborohydride.

Method C entails a 1,4-Michael addition reaction with an ethyl-4-oxo-4-arylcrotonate (e.g., XXV). The ketone oxygen of intermediate XXVII (i.e., Y is oxygen) is reduced by catalytic hydrogenation, preferably utilizing a palladium catalyst in the presence of small amounts of sulfuric acid to give XXVI.

Since the preferred embodiment of compounds of this invention relates to those compounds of formula I wherein R is H and $R_1$ is other than H (preferably ethyl) certain comments relating to the foregoing

coupling procedures of Reaction scheme D are to be noted, particularly as they relate to the selective hydrolysis of the R ester groups. For example, in general, for the selective hydrolysis of compounds XXVI it is preferred to prepare esters wherein R is a member of group (h) (i.e., diphenylmethyl, triphenylmethyl or benzyl) because these groups may be selectively hydrolysed with mild acids, e.g., by reaction with ethereal hydrochloric acid or with trifluoroacetic acid, or else they may be subjected to catalytic hydrogenolysis. Similarly, as the reduction of the ketonic oxygen (Y is O in formula XXVII) will also hydrolyze off the R group of the ester moiety to its corresponding acid it is also preferred that compounds XXVII have an ester group wherein R is selected from group (h). Also, peculiar to any compound wherein $R_3$ and $R_4$, together with the atoms to which they are attached, form a six membered ring, any R group (other than H) may be selectively hydrolyzed to its corresponding acid by treatment with a perfluoroalkyl sulfonic acid. Suitable perfluoro alkyl sulfonic acids are trifluoromethane sulfonic acid, pentafluoroethane sulfonic acid and heptafluoropropane sulfonic acid.

The following examples illustrate the techniques and conditions by which the compounds of this invention may be prepared. The preferred diastereomers of these examples are isolatable by conventional means.

## EXAMPLE 1

[2(S)]N-(2-Chloro-2-cyclohexene-1-yl)-1,3-dihydro-1,3-dioxo-2H-isoindole-2-(S)-[phenylmethyl-2-acetamide) (Xa)

**Step A.** 2-(2-chloro-2-chloro-2-cyclohexen-1-yl)-1H-isoindole-1,3(2H)-dione

A solution of 11.0g (72.8 mmol) 1,6-dichlorocyclohexane, 20.0g (108 mmol) potassium phthalimide and 1.0 g (6.0 mmol) potassium iodide in 50 ml dry dimethylformamide (DMF) was stirred 24 hours at 110°C under an atmosphere of nitrogen. The reaction mixture was allowed to cool then poured into 30 ml diethyl ether. The dark mixture was filtered then ether and DMF removed in vacuo. The dark crystalline residue was dissolved in ethyl acetate then chromatographed on 500 g flash silica eluting with 10% to 20% ethyl acetate-hexane. Concentration of appropriate fractions followed by recrystallization from ethyl acetate-hexane gave 14.0 g (73.5%) of the desired phthalimide, mp 99-103°C.

**Step B.** A solution of 6.0 g (120 mmol) of hydrazinehydrate and 26.1 g (100 mmol) of N-phthalimido-6-amino-1-chlorocyclohexene in 150 ml MeOH was refluxed under $N_2$ for 3 hrs., cooled to 25°C and allowed to stir for 3 hrs. The mixture was filtered, concentrated, poured into 300 ml 1N HCl, and washed with 200 ml $CH_2Cl_2$. The aqueous layer was basified and extracted with three 500 ml portions of $CH_2Cl_2$. The organics were dried over $MgSO_4$, filtered, and concentrated to give 9.25 g (70 mmol) of crude amine. The neutral extract was concentrated to give 6.0 g of unreacted starting phthalimide. To a stirred solution of 21 g (71 mmol) of phthalimido-L-phenylalanine and 18.5 g (75 mmol) of EEDQ in 200 ml $CH_2Cl_2$ at 25°C under $N_2$ was added 9.25 g (70 mmol) of the 6-amino-1-chlorocyclohexene in 20 ml $CH_2Cl_2$ over 30 min. After stirring for 18 hours, the reaction mixture was washed with two 200 ml portions of 10% HCl solution, 200 ml sat. $NaHCO_3$ solution, and brine. The organics were dried over $MgSO_4$, filtered and concentrated to give a solid. Recrystallization from $CH_2Cl_2$/Hexane gave 26.1 g of a mixture of the desired 2-(S) diastereomeric amides (Xa) which were not separated at this point. (64% overall yield): IR(KBr) 3400, 1775, 1715, 1650, 1530, 1380, $cm^{-1}$; NMR $\delta$ 1.60 (m,2H), 1.82 (m,2H), 2.05 (m,2H), 3.49 (s,1H), 3.59 (s,1H), 4.60 (m,1H), 5.05 (dd, 1/2H, $J_a = 10H_z$, $J_b = 2H_z$), 5.17 (dd, 1/2H, $J_a = 10H_z$, $J_b = 2H_z$), 5.95 (t, 1H, $J = 7\ H_z$), 6.45 (m,1H), 7.10 (s,5H), 7.70 (m,4H).

Anal. Calcd. for $C_{23}H_{21}ClN_2O_3$: C,67.56; H,5.18; N,6.85. Found: C,67.40; H,5.30; N,6.80.

## EXAMPLE 2

[S(R*,R*)]-1-[2-(1,3-Dihydro-1,3-dioxo-2H-isoindol-2-yl)-1-oxo-3-phenylpropyl]-1,2,3,4-tetrahydro-2-pyridine-carboxylicacid, methyl ester (XIa)

A solution of 12.2 g (30 mmol) of vinyl chlorides (Xa) from Example 1 in 300 ml $CH_2Cl_2$ containing 20 ml abs. MeOH was cooled to -70°C and stirred while a stream of ozone in oxygen (generated by a Welsbach Ozonator) was passed via a glass frit into the solution. When the solution turned blue, excess ozone was removed by passing dry $N_2$ into the solution. The reaction mixture was treated with 20 ml $Me_2S$ and 4 ml pyridine then allowed to gradually warm to 25°C and stir for 20 hours. The solution was poured into 200 ml 10% HCl solution and the organics were separated, washed well with $H_2O$, dried over $MgSO_4$

and concentrated to give 13.0 g of an amber oil. The crude ozonolysis product was dissolved in 200 ml $CH_2Cl_2$ containing 0.5 ml trifluoroacetic acid (TFA) and refluxed under $N_2$ for 3 hours. The cooled solution was washed with sat. $NaHCO_3$ solution, dried over $MgSO_4$ and concentrated to give 12.2 g of an amber oil. Preparative HPLC separation using 50% EtOAc/Hexane (Waters Prep-500 one recycle) gave 4.3 g (10.1 mmol) of each diastereomeric acylenamine XIa and XIb (n = 2), (68% overall yield). Isomer XIa was recrystallized from $CH_2Cl_2$/Hexane to give a fine white crystalline powder: mp 146-147 ° C;

$$[\alpha]_D^{Amb} = -320.1°$$

(c = 1.1, $CHCl_3$); IR (KBr) 1770, 1740, 1720, 1670, 1650, 1390, 1220, 722 $cm^{-1}$; NMR $\delta$1.85 (m,2H), 2.30 (m,2H), 3.50 (d, 2H, J = 7 $H_z$), 3.72 (s,3H), 4.71 (m,1H), 5.20 (m,1H), 5.27 (t, 1H, J = 7 $H_z$), 6.45 (d, 1H, J = 9 $H_z$), 7.12 (s,5H), 7.71 (m,4H).

Anal. Calcd. for $C_{23}C_{22}N_2O_5$: C,68.89; H,5.30; N,6.69. Found: C,68.61; H,5.26; N,6.56.

## EXAMPLE 3

[4S-(4α,7α,12bβ)]-7-(1,3-Dihydro-1,3-dioxo-2H-isoindo-2-yl)-1,2,3,4,6,7,8,12b-octahydro-6-oxopyrido[2,1-a]-[2]benzazepine-4-carboxylic acid, diphenylmethyl ester*(XIIa)

To a stirred solution of 4.20 g (10.0 mmol) of desired acylenamine XIa from Example 2 in 20 ml $CH_2Cl_2$ under $N_2$ atmosphere was added 6 ml $CF_3SO_3H$. After stirring at 20 ° C for 18 hours, the solution was poured onto ice and extracted into 200 ml EtOAc. The organics were washed well with water, dried over $MgSO_4$ and concentrated. The residue was dissolved in $CH_2Cl_2$ and treated with 2.2 g diazodiphenylmethane and allowed to stand for 12 hours. The solution was concentrated and the residue was flash chromatographed on 400 ml silica using 33% EtOAc/Hexane to give 4.5 g (7.7 mmol, 77% yield) of benzhydrylester XII (R = $CHPh_2$) as a foam. Recrystallization from $CH_2Cl_2$/Hexane was slow but gave 4.3 g (75% yield) of pure transparent plates: mp. 156-157 ° C;

$$[\alpha]_D^{Amb} = -87.6°$$

(c = 0.6, $CHCl_3$);IR 1780, 1717, 1643, 1450, 1379 $cm^{-1}$; NMR $\delta$ 1.8-2.1 (m,4H), 2.38 (m,2H); 3.23 (dd, 1H, $J_a$ = 18 $H_z$, $J_b$ = 16 $H_z$), 4.38 (dd, 1H, $J_a$ = 19 $H_z$, $J_b$ = 12 $H_z$), 5.30 (dd, 1H, $J_a$ = 6 $H_z$, $J_b$ = 2 $H_z$), 5.42 (dd, 1H, $J_a$ = 6 $H_z$, $J_b$ = 4 $H_z$), 6.05 (dd, 1H, $J_a$ = 12 $H_z$, $J_b$ = 6 $H_z$), 6.30 (s,1H), 6.61 (d, 1H, J = 7 $H_z$), 6.9-7.4 (m,13 H), 7.75 (m,2H), 7.92 (m,2H).

Anal. Calcd. for $C_{36}H_{30}N_2O_5$: C,75.77; H,5.30; N,4.91. Found: C,75.79; H,5.46; N,4.77.

## EXAMPLE 4

[4S-(4α,7α,12bß)]-7-(1,3-Dihydro-1,3-dioxo-2H-isoindol-2-yl)-1,2,3,4,6,7,8,12b-octahydro-6-oxopyrido[1-a][2]-benzazepine-4-carboxylic acid, methyl ester

Alternatively the cyclization product could be treated with diazomethane to give methyl ester XII from Example 3 (R = $CH_3$): mp 138-149 ° C;

$$[\alpha]_D^{Amb} = -122.4°$$

(c = 0.97, EtOH); IR 1778, 1720, 1655, 1620, 1375 $cm^{-1}$, NMR $\delta$ 1.7-2.2 (m,4H), 2.43 (m,2H), 3.10 (s,3H), 3.44 (dd, 1H, $J_a$ = 17 $H_z$, $J_b$ = 6 Hz), 4.42 (dd, 1H, $J_a$ = 17 $H_z$, $J_b$ = 12 $H_z$), 5.23 (dd, 1H, $J_a$ = 6 Hz, $J_b$ = 2 $H_z$), 5.47 (dd, 1H, $J_a$ = 6 $H_z$, $J_b$ = 4 $H_z$), 6.08 (dd, 1H, $J_a$ = 12 $H_z$, $J_b$ = 6 $H_z$), 7.23 (m,4H), 7.77 (m,2H), 7.89 (m,2H).

Anal. Cacd. for $C_{24}H_{22}N_2O_5$: C,68.89; H,5.30; N,6.69. Found: C,68.98; H,5.83; N,6.63.

## EXAMPLE 5

*(A compound of formula XII wherein R is diphenylmethyl and $R_5$ and $R_6$ are hydrogen and n is 3)

[4α,7α,12bβ)]-7-[[1-(Ethoxycarbonyl)-3-phenylpropyl]amino]-1,2,3,4,6,7,8,12b-octahydro-6-oxopyrido[2,1-a]-[2]benzazepine-4-carboxylic acid, diphenylmethyl ester

To a solution of 1.17 g (2.0 mmol) of phthalimide XII from Example 3 ($R = CHPh_2$) in 15 ml dry MeOH was added 2.3 ml 1N hydrazine hydrate solution in MeOH and the solution was stirred at 25°C for 3 days. The solvent was removed *in vacuo* giving a residue which was dissolved in $CHCl_3$, filtered and concentrated to give the crude amine as a light yellow oil. This crude amine (ca. 2.0 mmol) was dissolved in 6 ml $CH_2Cl_2$ under $N_2$ at 25°C and treated with 545 mg (2.5 mmol) 1,8-bis-(dimethylamino)naphthalene followed by 850 mg (2.5 mmol) of (R)-ethyl-4-phenyl-2-trifluoromethanesulfonyloxybutanoate (XXIII). The solution was stirred at 25 °C for 18 hours during which time a precipitate formed. The reaction mixture was placed directly on 100 ml of silica gel and flash chromotographed using 25% EtOAc/Hexane to give 1.11 g (1.76 mmol) 88% yield of pure S,S,S,R diester of the title as an oil: IR (KBr) 1734, 1657, 1495, 1452, 1185, 1155 $cm^{-1}$; NMR δ 1.28(t, 2H, J = 7 $H_z$), 1.7-2.2 (m,6H), 2.43 (m,2H), 2.68 (dd, 1H, $J_a$ = 17 $H_z$, $J_b$ = 13 $H_z$), 2.80 (m,2H), 3.25 (dd, 1H, $J_a$ = 17 $H_z$, $J_b$ = 6$H_z$), 3.46 (t, 1H, J = 7 $H_z$), 4.17 (q, 1H, J = 7 $H_z$), 4.38 (dd, 1H, $J_a$ = 13 $H_z$, $J_b$ = 6 $H_z$), 5.35 (dd, 1H, $J_a$ = 6 $H_z$, J = 4 $H_z$), 5.40 (dd, 1H, $J_a$ = 6 $H_z$, $J_b$ = 2 $H_z$), 6.25 (s, 1H).

## EXAMPLE 6

[4α,7α(R*),12bβ]-7-[[1-(Ethoxycarbonyl)-3-phenylpropyl]-amino]-1,2,3,4,6,7,12b-octahydro-6-oxopyrido[2,1-a][2]-benzazepine-4-carboxylic acid

To a stirred solution of 900 mg (1.42 mmol) of (S,S,S,R) benzhydryl ester of Example 5 ($R_3$,$R_4$ = -$CH_2CH_2CH_2$-, $R = CHPh_2$) and 2.5 ml of anisole at 25°C under $N_2$ was added 7 ml of trifluoroacetic acid. After stirring for 2 hours, the volatiles were removed in high vacuum to give an oily residue which was dissolved in 4 ml dry ether, stirred vigorously and diluted with hexane. The supernatant was decanted from the gummy solid which was triturated with hexane and vacuum dried to give 750 mg (1.3 mmol) of the tan solid TFA salt of (S,S,S,R) compound of the title in (91% yield): $[\alpha]^{Amb=}$ 25.5° (c = 0.57, $CH_3OH$); IR (KBr) 2300-3400, 1735, 1660, 1195, 1140 $cm^{-1}$; NMR δ ($CD_3CN$,TFA) 1.31 (t, 3H, J = 7 $H_z$), 1.78 (m,2H), 2.3-2.5 (m,4H), 2.84 (m,2H), 3.26 (dd, 1H, $J_a$ = 17 $H_z$, $J_b$ = 13 $H_z$), 3.68 (dd, 1H, $J_a$ = 17 $H_z$, $J_b$ = 6 $H_z$), 4.07 (t, 1H, J = 6 $H_z$), 4.29 (m,2H), 5.10 (dd, 1H, $J_a$ = 6 $H_z$, Jb = 2 Hz) 5.20 (dd, 1H, $J_a$ = 13 $H_z$, $J_b$ = 6 $H_z$), 5.35 (dd, 1H, $J_a$ = 5 $H_z$, $J_b$ = 1 $H_z$), 7.1-7.4 (m,9H).

Anal. Calcd. for $C_{29}H_{33}F_3N_2O_7$: C,60.20; H,5.75; N,4.84. Found: 6,60.12; H,5.72; N,4.45.

## EXAMPLE 7

[4α,7α(R*),12bβ]-7-[[1-carboxy-3-phenylpropyl]amino]-1,2,3,4,6,7,12b-octahydro-6-oxopyrido[2,1-a][2]-benzazepine-4-carboxylic acid

To a solution of 116 mg (0.20 mmol) of the ester of Example 6 in 5 ml 95% EtOH under $N_2$ at 25°C was added 0.50 ml 1N stock LiOH solution. After stirring for 18 hours, 0.50 ml 1N HCl was added in dropwise fashion with vigorous stirring. The zwitterion was isolated by filtration and vacuum dried to give 80 mg (0.17 mmol) 85% yield of a white solid which was homogeneous by analytical HPLC (Whatman Partisil 10 ODS-3 column, 0.1 M ammonium formate buffer in 50% MeOH/$H_2O$). Repeated analytical runs on a portion of the sample gave 8 mg of fine colorless crystals from the eluant buffer: mp 259-260°C(dec.);

$$[\alpha]_D^{Amb} = +24°$$

(c = 0.05,MeoH); IR(KBr) 1745, 1653, 1630, 1495, 1420, 1305, 1220, 752, 695 $cm^{-1}$; NMR ($CD_3CN$,TFA) δ 1.80 (m,4H), 2.3-2.4 (m,2H), 2.9 (m,2H), 3.29 (dd, 1H, $J_a$ = 17 $H_z$, $J_b$ = 13 $H_z$), 3.70 (dd, 1H, $J_a$ = 17 $H_z$, $J_b$ = 6 $H_z$), 4.13 (dd, 1H, $J_a$ = 10 $H_z$, $J_b$ = 5 Hz), 5.13 (dd, 1H, $J_a$ = 6 $H_z$, $J_b$ = 2 $H_z$), 5.24 (dd, 1H, $J_a$ = 13 $H_z$, $J_b$ = 6 $H_z$), 5.36 (dd, 1H, $J_a$ = 6 $H_z$, $J_b$ = 1 Hz), 7.2-7.4 (m,9H).

Anal. Calcd. for $C_{25}H_28N_2O_5$: C,68.79; H,6.46; N,6.42. Found: C,68.49; H,6.53; N,6.50.

## EXAMPLE 8

2-Amino-5-heptenoic acid, methyl ester

To a solution of 15.4 ml (110 mmol) of diisopropyl amine in 250 ml dry THF at -78° C was added 39 ml (105 mmol) 2.7 M n-butyl lithium in hexane. After stirring for 30 min., 20 ml hexamethylphosphoric triamide and a solution of 17.7 g (100 mmol) Schiff-base of benzaldehyde and glycine methyl ester in 25 ml THF were added over 30 min. After an additional 15 min., 13.5 g (100 mmol) 5-bromo-1-pentene was added and the solution was allowed to warm to 25° C slowly. After 3 hours, the reaction mixture was poured into water and extracted with ether. The extracts were repeatedly washed with brine, then dried over $MgSO_4$ and concentrated to give 25 g of an amber oil. This material was dissolved in 400 ml ether and stirred with 300 ml 0.5 N HCl for 2 hours. The aqueous layer was separated and the pH was adjusted to 9 with 1N NaOH. Extraction with chloroform, drying over $MgSO_4$, and concentration gave 4.5 g, as a liquid of the desired compound.

**EXAMPLE 9**

2-[[2-(1,3-dihydro-1,3-dioxo-2H-isoindol-2-yl)-1-oxo-3-phenylpropyl]amino]-6-heptenoic acid, methyl ester

To a solution of 6.0 g (20 mmol) phthalimido-L-phenylalanine and 6.0 g (24 mmol) EEDQ in 30 ml $CH_2Cl_2$ was added (21 mmol) of the product from Example 8 in 10 ml $CH_2Cl_2$. Gas evolution was observed and stirring was continued for 18 hours. The solution was diluted with $CH_2Cl_2$, washed with 10% HCl solution, sat. $NaHCO_3$ solution, and dried over $MgSO_4$. Concentration gave 8.3 g of a yellow oil which was flash chromatographed using 25% EtOAc/Hexane to give 6.0 g of diastereomeric amides Xb (n = 3) as a foam.

**EXAMPLE 10**

1,2,3,4-tetrahydro-1-[2-(1,3-dihydro-1,3-dioxo-2H-isoindol-2-yl)-1-oxo-3-phenylpropyl]-pyridine-2-carboxylic acid, methyl ester

Olefin from Example 9 (10 mmol) was dissolved in 100 ml $CH_2Cl_2$ containing 10 ml absolute MeOH and was cooled to -78° C. A stream of ozone in oxygen was passed into the stirred solution until a blue color persisted. After degassing with $N_2$, 10 ml dimethyl sulfide and 0.5 ml pyridine were added and the solution was allowed to warm slowly to 25° C and stir for 18 hours. The solution was washed with 3 portions 10% HCl solution, dried over $MgSO_4$ and concentrated to give an oil. This crude material was dissolved in 150 ml trichloroethane and treated with 0.5 ml trifluoroacetic acid at reflux for 18 hours. Concentration and flash chromatography gave chromatographically separable diastereomeric acylenamines XIa and XIb (n = 3).

**EXAMPLE 11**

7-(1,3-Dihydro-1,3-dioxo-2H-isoindo-2-yl)-1,2,3,4,6,7,8, 12b-octahydro-6-oxopyrido[2,1-a][2]benzazepine-4-carboxylic acid, diphenylmethyl ester

The desired acylenamine (1.6 mmol) (XIa) from Example 10 was dissolved in 5 ml $CH_2Cl_2$ and treated with 2.0 ml trifluoromethane sulfonic acid at 25° C under $N_2$ for 18 hours. The reaction mixture was partitioned between water and ethyl acetate. The organic extract was washed well with water, concentrated and treated with excess diazodiphenylmethane in $CH_2Cl_2$. Flash chromatography gave cyclized ester XIIa (n = 3, R = $CHPh_2$) as a foam.

**EXAMPLE 12**

[4R-[4α,7α(S),12bβ]]-7-[(1-carboxy-3-phenylpropyl)amino]-3,4,6,7,8,12b-hexahydro-6-oxo-1H-[1,4]thiazino-[3,4-a][2]-benzazepine-4-carboxylic acid

**Step A.** (R*,R*)-(-)-4-[2-(1,3-dihydro-1,3-dioxo-2H-isoindol-2-yl)-1-oxo-3-phenylpropyl]-3,4-dihydro-2H-1,4-thiazine-3-carboxylic acid, ethyl ester XVII

A 7.75 g (191 mmol) dispersion of 59% sodium hydride/paraffin was washed 2 times with 10 ml dry hexane under a stream of nitrogen. To the purified sodium hydride was added 90 ml dry DMF. To this mixture over 20 minutes under a stream of $N_2$ while cooling with an ice/MeOH bath was carefully added 17.9 g (96.7 mmol) of the HCl salt of serine ethyl ester. The mixture was allowed to stir 5 min. then 5.2 g

(32 mmol) potassium iodide was added. To this mixture was added 14.5 ml (96.7 mmol) bromoacetaldehyde diethyl acetal dropwise over 5 min. The cooling bath was then removed allowing the reaction temperature to rise to 30°C over the next 10 min. The mixture was allowed to stir 8 hrs. at ambient temperature then divided into 2 equal portions, one being added to a solution of 14.2 g (48 mmol) phthalimidophenylalanine and 11.9 g (48 mmol) EEDQ in 40 ml dry THF. The mixture was allowed to stir 18 hours at ambient temperature under $N_2$. The mixture was partitioned between 200 ml $H_2O$ and 200 ml diethyl ether. The phases were separated, and the aqueous portion was extracted with an additional 200 ml ether. The ethereal solutions were combined, successively treated with (a) 2 x 200 ml IN HCl, (b) 2 x 200 ml sat'd $NaHCO_3$, and (c) 50 ml brine. The so-extracted yellow ethereal solution was dried over $MgSO_4$, filtered, concentrated *in vacuo*, to yield 27.2 g of the expected acetal XVIa ($R = CH_2CH_3$, $X = S$) as an orange oil. To a solution of 16.1 g (30.3 mmol) of the acetal in 500 ml $CHCl_3$ was added 4.5 ml trifluoroacetic acid. The resultant solution was refluxed 4 hours under an atmosphere of $N_2$, cooled, extracted once with 300 ml saturated $NaHCO_3$, and filtered through anhydrous $MgSO_4$. The resultant solution was concentrated *in vacuo* to a dark foam which was chromatographed on 500 ml silica gel eluting with 1500 ml 35% EtOAc-hexane then with 55% EtOAc-hexane. The appropriate fractions were combined then concentrated to give 4.0 g (29%) of the acyl eneamine XVII ($R = -CH_2CH_3$, $X = S$) as a white foam which was crystallized from methanol to give analytically pure product as white needles. Mp 193°C,

$$[\alpha]_D^{Amb} = -375.5°$$

($c = 0.8, CHCl_3$); IR(KBr) 3400, 1770, 1740, 1720, 1680, 1620, 1380, 1180, 770, 690 cm-¹, 'H NMR $\delta$ (300 MHz); 1.28 (t, 3H, $J = 7.2$ Hz); 3.01 (dd, 1H, $J_a = 13.2$ Hz, $J_b = 3.1$ Hz); 3.36 (ddd, 1H, $J_a = 13.3$ Hz, $J_b = 3.1$ Hz, $J_c = 2.4$ Hz); 3.48 (d, 1H, $J = 2.6$ Hz); 3.50 (s,1H); 4.23 (q,2H, $J = 7.3$ Hz); 5.19 (dd, 1H, $J_a = 8.6$ Hz, $J_b = 2.1$ Hz); 5.33 (dd, 1H, $J_a = 8.9$ Hz, $J_b = 6.8$ Hz); 5.74 (t, 1H, $J = 3.1$ Hz); 5.57 (d, 1H, $J = 8.6$ Hz); 7.15 (s,5H); 7.71 (m,2H); 7.76 (m,2H). $^{13}C$ NMR $\delta$ (75.4 MHz): 14.1, 26.9, 35.0, 51.0, 53.3, 61.9, 101.9, 119.3, 123.4, 126.7, 128.3, 129.0, 130.9, 134.1, 136.3, 166.3, 166.8, 167.2.

Anal. Calcd. for $C_{24}H_{22}N_2O_5S$: C,63.99%; H,4.92%; N,6.22%. Found: C,64.07%; H,4.97%; N,6.20%.

**Step B.** [4S-(4α,7α,12bβ)]-7-(1,3-dihydro-1,3-dioxo-2H-isoindol-2-yl)-3,4,6,7,8,12b-hexahydro-6-oxo-1H-[1,4]-thiazino[3,4-a][2]benzazepine-4-carboxylic acid, methyl ester XVIII

A solution of 0.50 g (1.1 mmol) of the acyl enamine of Step A in 5 ml $CH_2Cl_2$ was added to 1.5 ml $CF_3SO_3H$ under an atmosphere of $N_2$ with stirring. The mixture was stirred at ambient temperature for 18 hours, then poured cautiously into a stirred suspension of excess $NaHCO_3$ in 10 ml methanol. The resultant mixture was concentrated *in vacuo*, partitioned between $CH_2Cl_2$ and $H_2O$ and the aqueous portion extracted with an additional portion of $CH_2Cl_2$. The organic portions were combined, dried over anhydrous $MgSO_4$, concentrated *in vacuo* to a yellow foam. The foam was dissolved in methanol, allowed to stand overnight at 0°C. The resultant crystals were collected, washed with cold methanol, then dried at ca. 0.5 mmHg at 60°C to give 0.35 g (72%) of the expected tricyclic ester XVIII ($R = CH_3$, $X = S$) as colorless needles, mp 130-134°C,

$$[\alpha]_D^{Amb} = -71.5°$$

($c = 0.4$, $CHCl_3$). IR(KBr): 3450, 1780, 1730, 1670, 1650,1380, 1300, 770, 720 $cm^{-1}$. 'H NMR $\delta$ (300 MHz): 2.93 (dd, 1H, $J_a = 13.6$ Hz, $J_b = 3.7$ Hz); 3.03 (dd, 1H, $J_a = 14.2$ Hz, $J_b = 4.0$ Hz); 3.26 (dd, 1H, $J_a = 16.4$ Hz, $J_b = 5.5$ Hz); 3.30 (s,3H); 3.38 (ddd, 1H, $J_a = 13.8$ Hz, $J_b = 5.8$ Hz, $J_c = 1.1$ Hz); 3.50 (dd, 1H, $J_a = 14.0$ Hz, $J_b = 6.7$ Hz); 4.40 (dd, 1H, $J_a = 16.5$ Hz, $J_b = 12.3$ Hz); 5.06 (t, 1H, $J = 4.3$ Hz); 5.39 (dd, 1H, $J_a = 6.0$ Hz, $J_b = 4.3$ Hz); 5.69 (dd, 1H, $J_a = 12.4$ Hz); 7.20-7.45(aromatic, 4H); 7.75 (m,2H); 7.88 (m,2H). $^{13}C$ NMR $\delta$ (75.4 MHz, proton decoupled): 27.3, 29.8, 33.9, 51.7, 57.0 (broad), 59.0 (broad), 123.4, 126.6, 127.1, 128.3, 130.2, 133.9, 135.9, 136.0, 167.8, 168.8, 169.2.

Anal. Calcd. for $C_{23}H_{20}N_2O_5S.H_2O$: C,60.78%; H,4.88%; N,6.16%; S,7.05%. Found: C,61.12%; H,4.71%; N,6.10%; S,7.07%.

**Step C.** [4R-(4α,7α,12bβ)]-7-amino-3,4,6,7,8,12b-hexahydro-6-oxo-1H-[1,4]thiazino[3,4-a][2]benzazepine-4-carboxylic acid, methyl ester

To a slurry of 0.67 g (1.5 mmol) of the tricyclic ester of Step B in 5 ml MeOH was added 3 ml (3.0 mmol) 1N hydrazine hydrate in MeOH. The mixture was allowed to stir at ambient temperature under an atmosphere of nitrogen for 60 hours. The mixture was then filtered through Celite with the cake being thoroughly washed with $CH_2Cl_2$. The filtrate was concentrated *in vacuo*, redissolved in $CH_2Cl_2$, and the organic solution was washed once with $H_2O$ then slowly filtered through $MgSO_4$. The filtrate was concentrated *in vacuo* to 419 mg of the desired amine as a yellow crystalline solid. An analytical sample was recrystallized from ethyl acetate-Hexane to give pure transparent needles mp 143° C. IR(KBr) 3420, 2900, 1730, 1715, 1660, 1430, 1370, 1320, 1300, 1270, 890, 760 $cm^{-1}$. NMR $\delta$ (300 MHz, $CDCl_3$) 1.82 (s,2H), 2.93 (dd, 1H, $J_a$ = 13.6 Hz, $J_b$ = 4.8 Hz), 2.97 (dd, 1H, $J_a$ = 16.2 Hz, $J_b$ = 13.2 Hz), 3.07 (s,3H), 3.19 (dd, 1H, $J_a$ = 14.5 Hz, $J_b$ = 5.0 Hz), 3.31 (ddd, 1H, $J_a$ = 14.5 Hz, $J_b$ = 3.8 Hz, $J_c$ = 2.1 Hz), 3.43 (dd, 1H, $J_a$ = 14.5 Hz, $J_b$ = 3.4 Hz), 3.44 (dd, 1H, $J_a$ = 17.4 Hz, $J_b$ = 6.2 Hz), 6.50 (dd, 1H, $J_a$ = 12.8 Hz, $J_b$ = 6.0 Hz), 5.56 (t, 1H, J = 4.4 Hz), 5.62 (dd, 1H, $J_a$ = 4.6 Hz, $J_b$ = 2.9 Hz), 7.10-7.25 (complex,3H), 7.37 (m,1H).

Anal. Calcd. for $C_{15}H_{18}N_2O_3S$: C,58.80; H,5.92; N,9.14. Found: C,58.70; H,5.97; N,9.00.

**Step D.** [4R-[4α,7α(S*),12bβ]]-7-[[1-(ethoxycarbonyl)-3-phenylpropyl]amino]-3,4,6,7,8,12b-hexahydro-6-oxo-1H-[1,4]thiazino[3,4-a][2]benzazepine-4-carboxylic acid, methyl ester

To a solution of 374 mg (1.22 mmol) of the amine of Step C and 282 mg (1.34 mmol) 1,8-bis-dimethylaminonaphthelene in 9 ml methylene chloride was added 457 mg (1.34 mmol) of the triflate of Example 5. The mixture was allowed to stir at ambient temperature under an atmosphere of nitrogen for 24 hours after which it was filtered. The filtrate was diluted with 10 ml 50% ethylacetate-hexane then filtered again. The resultant filtrate was concentrated *in vacuo* to a dark green glass. The glass was chromatographed on 150 ml silica eluting with 800 ml 37% ethyl acetate-hexane. Concentration *in vacuo* and drying yielded 514 mg (84.8%) of the desired diester as a white foam. IR(KBr): 3300, 2950, 2920, 1730, 1650, 1490, 1430, 1320, 1180, 910, 730, 690 $cm^{-1}$. 'H NMR $\delta$ (300 MHz): 1.22 (t, 3H, J = 7.0 Hz); 2.02 (m, 2H); 2.96 (s,3H); 3.18-3.47 (complex,5H); 4.12 (m, 2H); 4.41 (dd, 1H, $J_a$ = 13.0 Hz, $J_b$ = 6.0 Hz); 5.46 (t, 1H, J = 3.8 Hz); 5.52 (t, 1H, J = 3.2 Hz); 7.04-7.30 (aromatic, 9H.); $^{13}C$ NMR $\delta$ (75.4 MHz, proton decoupled): 14.4, 28.2, 28.4, 32.2, 35.0, 39.0, 50.0, 51.0, 51.9, 55.3, 60.3, 60.9, 125.3, 125.4, 125.9, 127.5, 128.3, 130.3, 134.9, 137.1, 141.1, 169.4, 174.1, 174.9. MS (chemical ionisation,methane): $MH^+$ = 497.3.

**Step E.** [4R-[4α,7α(S*),12bβ]]-7-[[1-(ethoxycarbonyl)-3-phenylpropyl]amino]-3,4,6,7,8,12b-hexahydro-6-oxo-1H-[1,4]thiazino[3,4-a][2]benzasepine-4-carboxylic acid

To 766 mg (1.54 mmol) of the diester of Step D was added 2.7 ml (31 mmol) trifluoromethane sulfonic acid at 15° C. The diester dissolved while cooling to 0° C. The dark solution was allowed to stir at 0°-5° C under an atmosphere of argon for 24 hours then poured cautiously into a solution of 4.03 g (46 mmol) $Na_2CO_3$ in 60 ml water. The resultant mixture was extracted with two 30 ml portions of ethyl acetate. The organic portions were discarded. The remaining aqueous solution was acidified to pH = 5 with aqueous hydrogen chloride. The resultant turbid mixture was extracted with three 60 ml portions of ethyl acetate. The organic portions were combined then washed with two 30 ml portions of brine. The organic solution was dried over anhydrous magnesium sulfate then concentrated *in vacuo* to give 288 mg (38%) of a yellow glass mp 138° C. Analytical data indicate the material to be composed of about 80% of the desired acid ester, the major contaminant being the sodium salt of trifluoromethane sulfonic acid. IR(KBr): 3420, 1730, 1650, 1500, 1430, 1250, 1160, 1030, 750, 690, 630 $cm^{-1}$. 'H NMR $\delta$ (300 MHz, $CD_3CN$): 1.23 (t, 3H, J = 7.1 Hz); 2.0 (m, 2H, from $CDCl_3$); 2.70 (t, 2H, J = 7.9 Hz); 2.86 (dd, 1H, $J_a$ = 17.5 Hz, $J_b$ = 12.2 Hz); 2.95 (dd, 1H, $J_a$ = 13.7 Hz, $J_b$ = 4.7 Hz); 3.17 (dd, 1H, $J_a$ = 14.7 Hz, $J_b$ = 4.9 Hz); 3.22-3.43 (complex,4H); 4.12(q,2H, J = 7.1 Hz); 4.54 (dd, 1H, $J_a$ = 12.9 Hz, $J_b$ = 5.7 Hz); 5.45 (dd, 1H, $J_a$ = 4.6 Hz, $J_b$ = 2.8 Hz); 5.60 (t, 1H, J = 4.0 Hz); 7.01-7.36 (aromatic, 9H.)

**Step F.** [4R-[4α,7α(S*),12bβ]]-7-[(1-carboxy-3-phenylpropyl)amino]-3,4,6,7,8,12b-hexahydro-6-oxo-1H-[1,4]-thiazino(3,4-a][2]-benzazepine-4-carboxylic acid

To a solution of 100 mg (0.2 mmol) of the diester of Step D in 2.27 ml methanol was added 0.5 ml (0.5 mmol) 1N lithium hydroxide. The solution clouded momentarily, but quickly became homogeneous with stirring. The solution was allowed to stir 60 hours at ambient temperature under an atmosphere of nitrogen. The solution was concentrated *in vacuo* to obtain a white residue. One half of the residue was purified by HPLC on a 25 cm x 22 mm ID Partisil 10-ODS3 column with a mobile phase of 0.1 M pH = 6.2 ammonium formate in 40% methanol-water. The first major peak was collected. The appropriate fractions were

combined then concentrated *in vacuo*. Residual ammonium formate was removed by Kugelrohr distillation at 90°C/1mm Hg to yield 13 mg (26%) of the desired diacid, mp 232-235°C (dec.) IR(KBr): 3420, broad 3100-2200, 1720, 1650, 1630, 1490, 1400, 1200, 750, 690 $cm^{-1}$. 'H NMR δ (300 MHz, $D_2O$-TFA): 2.35 (m,2H); 2.89 (td, 2H, $J_a$=10.3 Hz, $J_b$=6.7 Hz); 3.01 (dd, 1H, $J_a$=14.0 Hz, $J_b$=4.7 Hz); 3.23 (dd, 1H, $J_a$=15.0 Hz, $J_b$=4.8 Hz); 3.33 (d, 1H, 3=2.6 Hz); 3.39 (d, 1H, 3=2.6 Hz); 3.42 (dd, 1H, $J_a$=15.0 Hz, $J_b$=5.8 Hz); 3.71 (dd, 1H, $J_a$=16.1 Hz, $J_b$=6.3 Hz); 4.10 (broad, s,1H); 5.31 (broad, 1H); 5.48 (d, 1H, J=4.2 Hz); 5.52 (d, 1H, J=4.2 Hz); 7.16-7.38 (aromatic, 8H); 7.49 (d, 1H, J=8.3 Hz); 7.88 (broad, d, 2H, J=27.0 Hz).

The compounds of this invention form salts with various inorganic and organic acids and bases which are also within the scope of the invention. Such salts include ammonium salts, alkali metal salts like sodium and potassium salts, alkaline earth metal salts like the calcium and magnesium salts, salts with organic bases, e.g., dicyclohexylamine salts, N-methyl-D-glucamine, salts with amino acids like arginine, lysine and the like. Also salts with organic and inorganic acids can be prepared, e.g., HCl, HBr, $H_2CO_3$, $H_3PO_4$, methanesulfonic, toluenesulfonic, maleic, fumaric, camphorsulfonic. The non-toxic physiologically acceptable salts are preferred, although other salts are also useful, e.g., in isolating or purifying the product.

The salts can be formed by conventional means as by reacting the free acid or free base forms of the product with one or more equivalents of the appropriate base or acid in a solvent or medium in which the salt is insoluble, or in a solvent such as water which is then removed *in vacuo* or by freeze-drying or by exchanging the cations of an existing salt for another cation on a suitable ion exchange resin.

The compounds of this invention inhibit angiotensin converting enzyme and thus block conversion of the decapeptide angiotensin I to angiotensin II. Angiotensin II is a potent pressor substance. Thus, blood pressure lowering can result from inhibition of its biosynthesis especially in animals and humans whose hypertension is angiotensin II related. Furthermore, converting enzyme inhibitors may lower blood pressure also by potentiation of bradykinin. Although the relative importance of these and other possible mechanisms remains to be established, inhibitors of angiotensin converting enzyme are effective antihypertensive agents in a variety of animal models and are useful clinically, for example, in many human patients with renovascular, malignant and essential hypertension. See, for example, D.W. Cushman, et al., Biochemistry 16, 5484 (1977).

The evaluation of converting enzyme inhibitors is guided by *in vitro* enzyme inhibition assays. For example, a useful method is that of Y. Piquilloud, A. Reinharz and M. Roth, Biochem. Biophys. Acta, 206 N36 (1970) in which the hydrolysis of carbobenzyloxyphenylalanylhistidinyl-leucine is measured. *In vivo* evaluations may be made, for example, in normotensive rats challenged with angiotension I by the technique of J.R. Weeks and J.A. Jones, Proc. Soc. Exp. Biol. Med., 104, 646 (1960) or in a high renin rat model such as that of S. Koletsky et al., Proc. Soc. Exp. Biol. Med. 125, 96 (1967).

Thus, the compounds of this invention are useful as antihypertensives in treating hypertensive mammals, including humans, and they can be utilized to achieve the reduction of blood pressure by formulating them in appropriate compositions for administration. The compounds of this invention can be administered to patients in need of such treatment in a dosage range of 0.5 to 100 mg per patient generally given several times a day, thus giving a total daily dose of from 0.5 to 400 mg per day. The dose will vary depending on severity of disease, weight of patient and other factors which a person skilled in the art will recognize.

Thus, in accordance with the present invention there is provided a pharmaceutical composition for inhibiting angiotensin converting enzyme or treating hypertension comprising a pharmaceutically acceptable carrier and a pharmaceutically effective amount of a compound of Formula I.

For administration, the compositions of the invention can also contain other conventional pharmaceutically acceptable compounding ingredients, as necessary or desired. Such ingredients are generally referred to as carriers or diluents. Conventional procedures for preparing such compositions in appropriate dosage forms can be utilized. Whatever the dosage form, it will contain a pharmaceutically effective amount of the compound of the invention.

The present compositions can be administered orally or other than orally, e.g., parenterally, by insufflation, topically, rectally, etc.; using appropriate dosage forms; e.g., tablets, capsules, suspensions, solutions, and the like, for oral administration; suspensions, emulsions, and the like, for parenteral administration; solutions for intravenous administration; the ointments, transdermal patches, and the like, for topical administration.

Compositions intended for oral use may be prepared according to any method known to the art for the manufacture of pharmaceutical compositions and such compositions may contain one or more agents such as sweetening agents, flavoring agents, coloring agents and preserving agents in order to provide pharmaceutically elegant and palatable preparation. Tablets containing the active ingredient in admixture

with non-toxic pharmaceutically acceptable excipients may also be manufactured by known methods. The excipients used may be, for example, (1) inert diluents such as calcium carbonate, sodium carbonate, lactose, calcium phosphate or sodium phosphate; (2) granulating and disintegrating agents such as corn starch, or alginic acid (3) binding agents such as starch, or gelatin or acacia, and (4) lubricating agents such as magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Pat. No. 4,256,108; 4,160,452; and 4,265,874 to form osmotic therapeutic tablets for controlled release.

In some cases, formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, for example, peanut oil, liquid paraffin, or olive oil.

Aqueous suspensions normally contain the active materials in admixture with excipients suitable for the manufacture of aqueous suspensions. Such excipients may be

(1) suspending agents such as sodium carboxmethylcellulose, methylcellulose, hydroxypropylmethylcellulose, sodium alginate, polyvinylpyrrolidone, gum tragacanth and gum acacia;

(2) dispersing or wetting agents which may be

(a) a naturally-occurring phosphatide such as lecithin,

(b) a condensation product of an alkylene oxide with a fatty acid, for example, polyoxyethylene stearate,

(c) a condensation product of ethylene oxide with a long chain aliphatic alcohol, for example, heptadecaethyleneoxycetanol,

(d) a condensation product of ethylene oxide with a partial ester derived from a fatty acid and a hexitol such as polyoxyethylene sorbitol monooleate, or

(e) a condensation product of an ethylene oxide with a partial ester derived from a fatty acid and a hexitol anhydride, for example, polyoxyethylene sorbitan monooleate.

The aqueous suspensions may also contain one or more preservatives, for example, ethyl or n-propyl p-hydroxybenzoate; one or more coloring agents; one or more flavoring agents; and one or more sweetening agents such as sucrose or saccharin.

Oily suspension may be formulated by suspending the active ingredient in a vegetable oil, for example, arachis oil, olive oil, sesame oil or coconut oil, or in a mineral oil such as liquid paraffin. The oily suspensions may contain a thickening agent, for example, beeswax, hard paraffin or cetyl alcohol. Sweetening agents and flavoring agents may be added to provide a palatable oral preparation. These compositions may be preserved by the addition of an antioxidant such as ascorbic acid.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example, those sweetening, flavoring and coloring agents described above may also be present.

The pharmaceutical compositions of the invention may also be in the form of oil-in-water emulsions. The oily phase may be vegetable oil such as liquid paraffin or a mixture thereof. Suitable emulsifying agents may be (1) naturally-occurring gums such as gum acacia and gum tragacanth, (2) naturally-occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Syrups and elixirs may be formulated with sweetening agents, for example, glycerol, propylene glycol, sorbitol or sucrose. Such formulations may also contain a demulcent, a preservative and flavoring and coloring agents.

The pharmaceutical compositions may be in the form of a sterile injectable aqueous or oleagenous suspension. This suspension may be formulated according to known methods using those suitable dispersing or wetting agents and suspending agents which have been mentioned above. The sterile injectable preparation may also be a sterile injectable solution or suspension in a non-toxic parenterally-acceptable diluent or solvent, for example, vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

A composition of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compositions of the invention are employed.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The compounds of this invention can also be administered in combination with other antihypertensives and/or diuretics and/or calcium entry blockers. For example, the compounds of this invention can be given in combination with such compounds as acetazolamide, benzthiazide, bumetanide, chlorothalidone, chlorothiazide, clonidine, cryptenamine acetates and cryptenamine tannates, cyclothiazide, deserpidine, diazoxide, diltiazem, (S)-1-[[2-(3,4-dimethoxyphenyl)-ethyl]amino]-3-[4-(2-thienyl)-1H-imidazol-2-yl]phenoxy]-2-propanol, ethacrynic acid, flumethiazide, furosemide, guanethidine sulfate, hydralazine hydrochloride, hydrochlorothiazide, hydroflumethiazide, (+)-4-[3-[-(2-(1-hydroxycyclohexyl)-ethyl]-4-oxo-2-thiazolidinyl]-propyl]-benzoic acid, indacrinone and variable ratios of its enantiomers, merethoxylline procaine, methylclothiazide, methyldopa, methyldopate hydrochloride, metolazone, metroprolol tartrate, minoxidil, naldolol, pargyline hydrochloride, pindolol, polythiazide, prazosine, propranolol, quinethazone, rauwolfia serpentina, rescinnamine, reserpine, sodium ethacrynate, sodium nitroprusside, spironolactone, ticrynafen, timolol, triamterene, trichlormethiazide, trimethophan camsylate, bepridil, diltiazem, etafenone, falipamil, felodipine, flunarizine, gallopamil, indapamide, lidoflazine, nicardipine, nifedipine, nimopidine, nitrendipine, perhexiline, prenylamine, tiapamil, verapamil, and the like, as well as admixtures and combinations thereof.

Typically, the individual daily dosages for these combinations can range from about one fifth of the minimally recommended clinical dosages to the maximum recommended levels for the entities when they are given singly.

To illustrate these combinations, one of the antihypertensives of this invention effective in the 0.5 to 1000 mg per day range can be effectively combined with the following compounds at the indicated per day dose range: hydrochlorothiazide (10-100 mg); chlorothiazide (125-2000 mg); manipulated indacrinone enantiomer ratio (25-150 mg); ethacrynic acid (15-2000 mg); amiloride (5-20 mg); furosemide (5-80 mg); propranolol (20-480 mg); timolol (5-60 mg); and methyldopa (65-2000 mg); and the pivaloyloxyethyl ester of methyldopa (30-1000 mg). In addition, triple drug combinations of hydrochlorothiazide (10-100 mg) plus amiloride (5-20 mg) plus converting enzyme inhibitor of this invention (0.5-1000 mg) or manipulated indacrinone enantiomer ratio (25-150 mg) plus amiloride (5-20 mg) plus converting enzyme inhibitor of this invention (0.5-1000 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

With the term "conjunctive administration" it is intended that the active sustances may be administered simultaneously, in the same or in a separated pharmaceutical formulation, by the same or a different administration route, or may be administered one after the other by the same or a different administration route, at a time interval such that the pharmacological effects of the substance administered first are still present when the subsequent one begins its pharmacological action .

Preferred subgeneric groups of compounds are those of formula

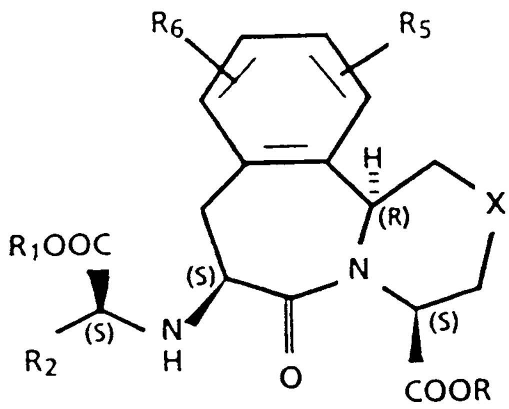

wherein $R_2$ is phenethyl,
wherein $R_5$ is H or hydroxy,
wherein $R_6$ is H or hydroxy,
wherein R is $C_{1-6}$ alkyl,
wherein $R_1$ is ethyl,
wherein R is H,
wherein X is $CH_2$, and
wherein X is S.

Preferred species are those compounds of the foregoing formulae with the specific values for R, $R_1$, $R_2$, X, $R_5$, $R_6$ being in accordance with the following table.

| R | $R_1$ | $R_2$ | X | $R_5$ | $R_6$ |
|---|---|---|---|---|---|
| H | Et | Phenethyl | $CH_2$ | H | H |
| H | Et | Phenethyl | $CH_2$ | OH | H |
| H | Et | Phenethyl | $CH_2$ | OH | OH |
| H | Et | Phenethyl | S | H | H |
| H | Et | Phenethyl | O | H | H |
| H | Et | Phenethyl | S | OH | H |

## Claims

**Claims for the following Contracting States : BE, CH, DE, FR, GB, IT, LU, NL, SE**

**1.** Compounds of the formula

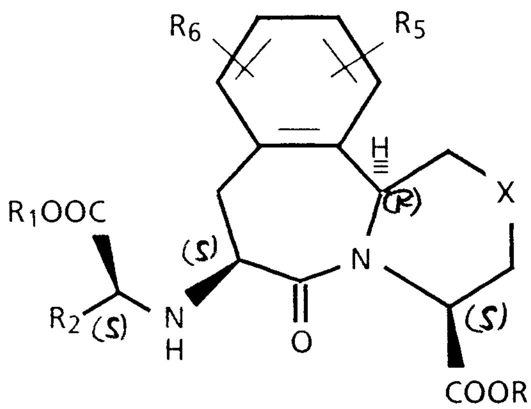

(I)

and the pharmaceutically acceptable salts thereof wherein

R and $R_1$ are independently

(a) hydrogen;

(b) $C_1$-$C_6$ alkyl;

(c) substituted $C_1$-$C_6$ alkyl wherein the substituents are hydroxy, $C_1$-$C_4$ alkyloxy and di-($C_1$-$C_4$)-alkylamino;

(d) $C_6$ to $C_{12}$ aryl;

(e) substituted $C_6$ to $C_{12}$ aryl wherein the substituents are $C_1$-$C_6$ alkyl, halo (F, Cl, Br, I)and $C_1$-$C_4$ alkyloxy;

(f) hetero ($C_4$ to $C_9$)aryl wherein the heteroatom can be one of O, N or S;

(g) substituted hetero ($C_4$ to $C_9$)aryl wherein the heteroatom can be one of O, N or S and the substituents are $C_1$-$C_6$ alkyl, halo (F, $C_1$, Br, I) and $C_1$-$C_4$ alkyloxy;

$R_2$ is

(a) hydrogen;

(b) $C_1$-$C_8$ straight or branched alkyl;

(c) $C_2$-$C_8$ straight or branched alkenyl;

(d) $C_2$-$C_8$ straight or branched alkynyl;

(e) $C_3$-$C_{10}$ cycloalkyl;

(f) $C_6$ or $C_{10}$ aryl ($C_1$-$C_4$)alkyl;

(g) substituted $C_1$-$C_8$ alkyl which contains a $CONR_2$, $SO_2NR_2$, $NRCONR_2$, $OCONR_2$, NRCOOR or $NR_2$ group, wherein R is as defined above;

$R_5$ and $R_6$ each independently are

(a) hydrogen, hydroxy;

(b) halo (F, Br, Cl, I);

(c) $C_1$-$C_6$ alkyl;

(d) $C_1$-$C_6$ alkyloxy; and X is $CH_2$, O or S.

**2.** A compound of claim 1 wherein $R_5$ and $R_6$ are hydrogen.

**3.** A compound of claim 1 wherein $R_5$ is hydroxy and $R_6$ is hydrogen.

**4.** A compound of claim 1 wherein R is H.

**5.** A compound of claim 4 wherein $R_2$ is phenethyl.

**6.** A compound of claim 5 wherein X is $CH_2$.

**7.** A compound of claim 6 wherein $R_1$ is ethyl.

**8.** A compound of claim 7 wherein X is S.

**9.** A compound of claim 7 wherein X is O.

**10.** A compound of claim 1 wherein R is H, $R_1$ is ethyl, X is $CH_2$, $R_2$ is phenethyl, $R_5$ and $R_6$ are H.

**11.** A compound of claim 1 wherein R is H, $R_1$ is ethyl, X is S, $R_2$ is phenethyl, and $R_5$ and $R_6$ are H.

**12.** A compound of claim 1 wherein R and $R_1$ are H, X is $CH_2$, $R_5$ and $R_6$ are H and $R_2$ is phenethyl.

**13.** A compound of claim 1 wherein R and $R_1$ are ethyl, X is $CH_2$, $R_5$ and $R_6$ are H and $R_2$ is phenethyl.

**14.** A compound of claim 1 wherein R and $R_1$ are H, X is S, $R_5$ and $R_6$ are H and $R_2$ is phenethyl.

**15.** A compound of claim 1 wherein R and $R_1$ are ethyl, X is S, $R_5$ and $R_6$ are H and $R_2$ is phenethyl.

**16.** A process for preparing compounds of formula I of claim 1 and the pharmaceutically acceptable salts thereof which comprises coupling a fused lactam of the formula

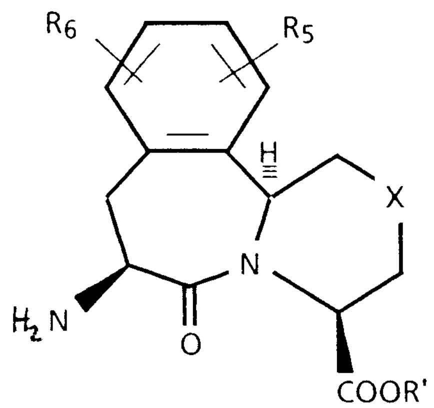

with a reactant of the formulae

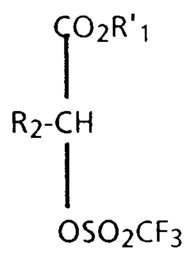

A

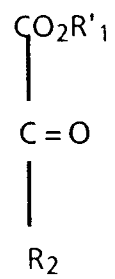

B

or

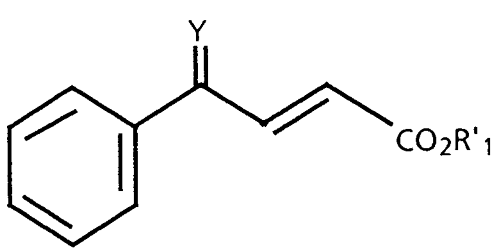

C

wherein R' and $R'_1$ have the same meanings as R and $R_1$ in Claim 1 with the exception of hydrogen, $R_2$, $R_5$, $R_6$ and X have the same meanings as in Claim 1, and Y is oxygen, and optionally removing one or both of the $R'_1$ and R' ester functions once said coupling has taken place, said coupling with a compound of formula B being effected by contacting the reactants together in the presence of a molecular sieve to form a Schiff's base and reducing said Schiff's base preferably with sodium cyanoborohydride, said coupling with a compound of formula C being effected according to a 1,4-Michael addition reaction, followed by the reduction of Y using catalytic hydrogenation techniques.

**17.** A process for preparing compounds of the formula

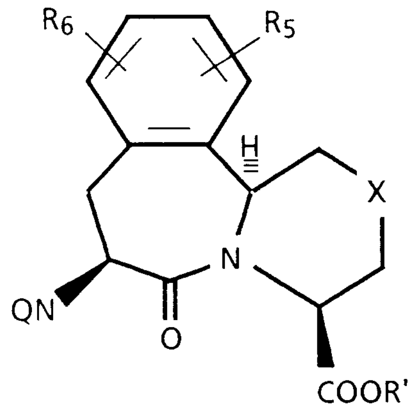

wherein Q is (H,H) or a N-protecting group, R' is as defined in claim 16 and X is $CH_2$, S, or O which comprises subjecting a compound of the formula

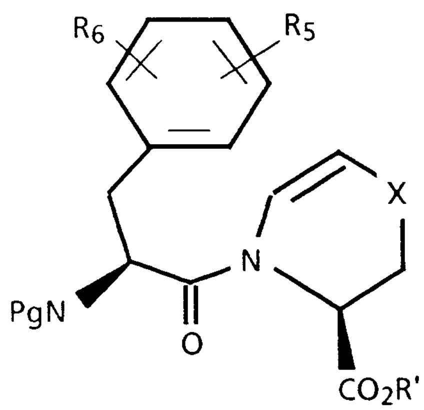

to a Friedel-Crafts cyclization, and optionally removing any N-protecting group, said cyclization being effected with a Lewis acid.

**18.** A process of claim 17 wherein the Lewis acid is a perfluoroalkylsulfonic acid.

**19.** A process of claim 17 wherein Q is phthalimido.

**20.** A process of claim 17 wherein the phthalimido protecting group is removed by reaction with hydrazine hydrate.

**21.** A compound of claim 1 to 15 for use as a medicine.

**22.** Use of a compound of claim 1 to 15 for preparing a medicament for treating hypertension.

**23.** A pharmaceutical composition which comprises a compound of claims 1 to 15 in admixture with a pharmaceutically acceptable carrier.

**24.** A medicament which comprises a compound of claims 1 to 15 for conjunctive administration with another antihypertensive and/or diuretic and/or calcium entry blocker.

**25.** A pharmaceutical composition which comprises a compound of claims 1 to 15 and one or two other active ingredients selected from another antihypertensive, a diuretic and a calcium entry blocker.

**Claims for the following Contracting States : AT, ES, GR**

**1.** A process for preparing compounds of the formula

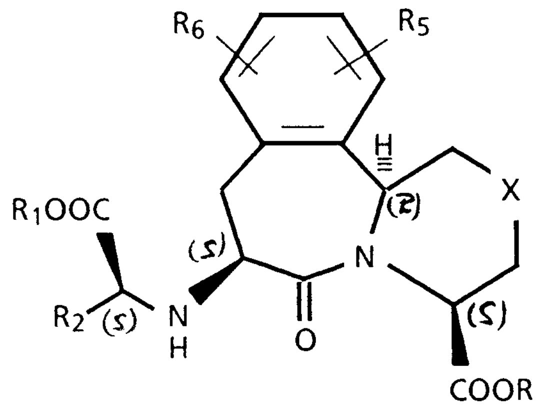

(I)

and the pharmaceutically acceptable salts thereof wherein
R and $R_1$ are independently
(a) hydrogen;
(b) $C_1$-$C_6$ alkyl;
(c) substituted $C_1$-$C_6$ alkyl wherein the substituents are hydroxy, $C_1$-$C_4$ alkyloxy and di-($C_1$-$C_4$)-alkylamino;
(d) $C_6$ to $C_{12}$ aryl;
(e) substituted $C_6$ to $C_{12}$ aryl wherein the substituents are $C_1$-$C_6$ alkyl, halo (F, Cl, Br, I)and $C_1$-$C_4$ alkyloxy;
(f) hetero ($C_4$ to $C_9$)aryl wherein the heteroatom can be one of O, N or S;
(g) substituted hetero ($C_4$ to $C_9$)aryl wherein the heteroatom can be one of O, N or S and the substituents are $C_1$-$C_6$ alkyl, halo (F, $C_1$, Br, I) and $C_1$-$C_4$ alkyloxy;
$R_2$ is
(a) hydrogen;
(b) $C_1$-$C_8$ straight or branched alkyl;
(c) $C_2$-$C_8$ straight or branched alkenyl;
(d) $C_2$-$C_8$ straight or branched alkynyl;
(e) $C_3$-$C_{10}$ cycloalkyl;
(f) $C_6$ or $C_{10}$ aryl ($C_1$-$C_4$)alkyl;
(g) substituted $C_1$-$C_8$ alkyl which contains a $CONR_2$, $SO_2NR$, $NRCONR_2$, $OCONR_2$, NRCOOR or $NR_2$ group, wherein R is as defined above;
$R_5$ and $R_6$ each independently are
(a) hydrogen, hydroxy;
(b) halo (F, Br, Cl, I);
(c) $C_1$-$C_6$ alkyl;
(d) $C_1$-$C_6$ alkyloxy; and X is $CH_2$, O or S, which comprises coupling a fused lactam of the formula

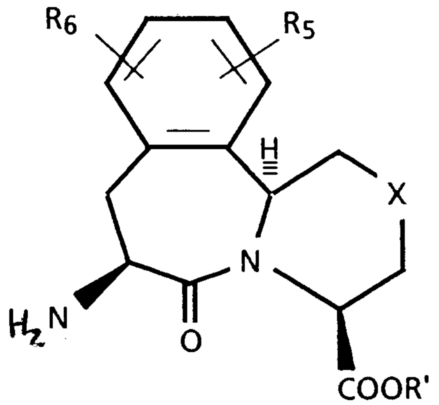

with a reactant of the formulae

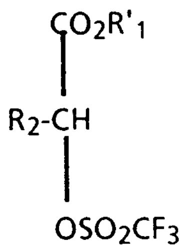 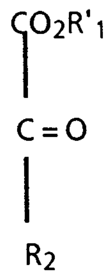 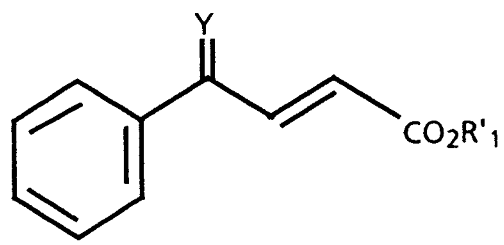

A B or C

wherein R' and $R'_1$ have the same meanings as R and $R_1$ above with the exception of hydrogen, $R_2$, $R_5$, $R_6$ and X have the same meanings as above, and Y is oxygen and optionally removing one or both of the $R'_1$ and R' ester functions once said coupling has taken place, said coupling with a compound of formula B being effected by contacting the reactants together in the presence of a molecular sieve to form a Schiff's base and reducing said Schiff's base preferably with sodium cyanoborohydride, said coupling with a compound of formula C being effected according to a 1,4-Michael addition reaction, followed by the reduction of Y using catalytic hydrogenation techniques.

**2.** A process for preparing compounds of the formula

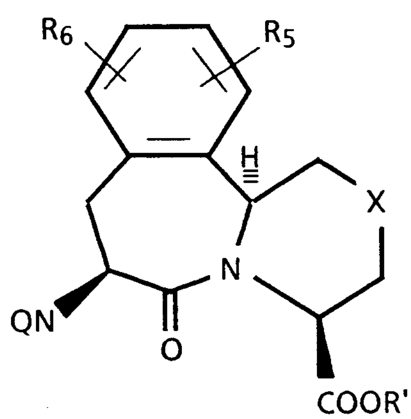

wherein Q is (H,H) or a N-protecting group, R' is as defined in Claim 1 and X is $CH_2$, S, or O which comprises subjecting a compound of the formula

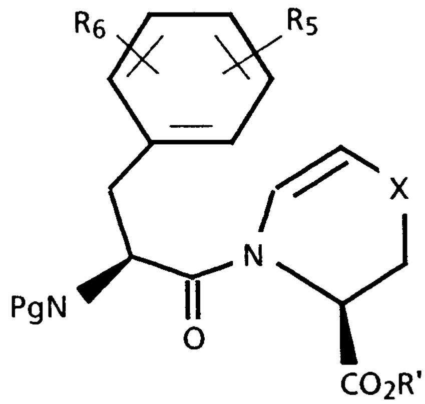

wherein $P_9$ is a N-protecting group, to a Friedel-Crafts cyclization, and optionally removing any N-protecting group, said cyclization being effected with a Lewis acid.

3. A process of claim 2 wherein the Lewis acid is a perfluoroalkylsulfonic acid.

4. A process of claim 2 wherein Q is phthalimido.

5. A process of claim 4 wherein the phthalimido protecting group is removed by reaction with hydrazine hydrate.

6. A process according to claims 1 to 5 for preparing a compound wherein $R_5$ and $R_6$ are hydrogen.

7. A process according to claims 1 to 5 for preparing a compound wherein $R_5$ is hydroxy and $R_6$ is hydrogen.

8. A process according to claims 1 to 5 for preparing a compound wherein R is H.

9. A process according to claims 1 to 5 for preparing a compound wherein $R_2$ is phenethyl and R is hydrogen.

10. A process according to claims 1 to 5 for preparing a compound wherein X is $CH_2$, R is H and $R_2$ is phenethyl.

11. A process according to claims 1 to 5 for preparing a compound wherein $R_1$ is ethyl, R is H, $R_2$ is phenethyl and X is $CH_2$.

12. A process according to claims 1 to 5 for preparing a compound wherein X is S, R is H, $R_1$ is ethyl and $R_2$ is phenethyl.

13. A process according to claims 1 to 5 for preparing a compound wherein X is O, R is H, $R_1$ is ethyl and $R_2$ is phenethyl.

14. A process according to claims 1 to 5 for preparing a compound wherein R is H, $R_1$ is ethyl, X is $CH_2$, $R_2$ is phenethyl, $R_5$ and $R_6$ are H.

15. A process according to claims 1 to 5 for preparing a compound wherein R is H, $R_1$ is ethyl, X is S, $R_2$ is phenethyl, and $R_5$ and $R_6$ are H.

16. A process according to claims 1 to 5 for preparing a compound wherein R and $R_1$ are H, X is $CH_2$, $R_5$ and $R_6$ are H and $R_2$ is phenethyl.

17. A process according to claims 1 to 5 for preparing a compound wherein R and $R_1$ are ethyl, X is $CH_2$, $R_5$ and $R_6$ are H and $R_2$ is phenethyl.

**18.** A process according to claims 1 to 5 for preparing a compound wherein R and $R_1$ are H, X is S, $R_5$ and $R_6$ are H and $R_2$ is phenethyl.

**19.** A process according to claims 1 to 5 for preparing a compound wherein R and $R_1$ are ethyl, X is S, $R_5$ and $R_6$ are H and $R_2$ is phenethyl.

**20.** Use of a compound of any of the above claims for preparing a medicament for treating hypertension.

**Patentansprüche**
**Patentansprüche für folgende Vertragsstaaten : BE, CH, DE, FR, GB, IT, LU, NL, SE**

**1.** Verbindungen der Formel

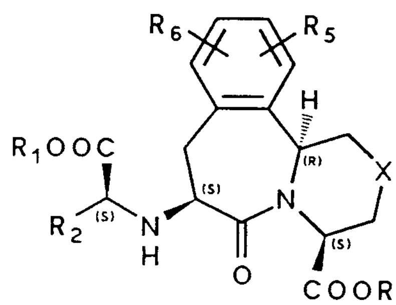

(I)

und die pharmazeutisch verträglichen Salze davon, wobei
R und $R_1$ unabhängig voneinander
(a) ein Wasserstoffatom;
(b) einen $C_1$-$C_6$-Alkylrest;
(c) einen substituierten $C_1$-$C_6$-Alkylrest, in dem die Substituenten Hydroxylgruppen, $C_1$-$C_4$-Alkyloxy- und Di-($C_1$-$C_4$)-alkylaminoreste sind;
(d) einen $C_6$-$C_{12}$-Arylrest;
(e) einen substituierten $C_6$-$C_{12}$-Arylrest, in dem die Substituenten $C_1$-$C_6$-Alkylreste, Halogenatome (F, Cl, Br, I) und $C_1$-$C_4$-Alkyloxyreste sind;
(f) einen Hetero-($C_4$-$C_9$)-arylrest, in dem das Heteroatom ein Sauerstoff-, Stickstoff- oder Schwefelatom sein kann;
(g) einen substituierten Hetero-($C_4$-$C_9$)-arylrest, in dem das Heteroatom ein Sauerstoff-, Stickstoff- oder Schwefelatom sein kann, und die Substituenten $C_1$-$C_6$-Alkylreste, Halogenatome (F, Cl, Br, I) und $C_1$-$C_4$-Alkyloxyreste sind,
bedeuten;
$R_2$
(a) ein Wasserstoffatom;
(b) einen unverzweigten oder verzweigten $C_1$-$C_8$-Alkylrest;
(c) einen unverzweigten oder verzweigten $C_2$-$C_8$-Alkenylrest;
(d) einen unverzweigten oder verzweigten $C_2$-$C_8$-Alkinylrest;
(e) einen $C_3$-$C_{10}$-Cycloalkylrest;
(f) einen $C_6$- oder $C_{10}$-Aryl-($C_1$-$C_4$)-alkylrest;
(g) einen substituierten $C_1$-$C_8$-Alkylrest, der einen Rest der Formel $CONR_2$, $SO_2NR_2$, $NRCONR_2$, $OCONR_2$, $NRCOOR$ oder $NR_2$ enthält, wobei R wie vorstehend definiert ist,
bedeutet;
$R_5$ und $R_6$ jeweils unabhängig voneinander
(a) ein Wasserstoffatom, eine Hydroxylgruppe;
(b) ein Halogenatom (F, Br, Cl, I);
(c) einen $C_1$-$C_6$-Alkylrest;
(d) einen $C_1$-$C_6$-Alkyloxyrest
bedeuten;

und X ein Rest der Formel $CH_2$, ein Sauerstoff- oder Schwefelatom ist.

2. Verbindung nach Anspruch 1, wobei $R_5$ und $R_6$ ein Wasserstoffatom bedeuten.

3. Verbindung nach Anspruch 1, wobei $R_5$ eine Hydroxylgruppe und $R_6$ ein Wasserstoffatom bedeuten.

4. Verbindung nach Anspruch 1, wobei R ein Wasserstoffatom bedeutet.

5. Verbindung nach Anspruch 4, wobei $R_2$ eine Phenethylgruppe bedeutet.

6. Verbindung nach Anspruch 5, wobei X einen Rest der Formel $CH_2$ bedeutet.

7. Verbindung nach Anspruch 6, wobei $R_1$ eine Ethylgruppe bedeutet.

8. Verbindung nach Anspruch 7, wobei X ein Schwefelatom bedeutet.

9. Verbindung nach Anspruch 7, wobei X ein Sauerstoffatom bedeutet.

10. Verbindung nach Anspruch 1, wobei R ein Wasserstoffatom, $R_1$ eine Ethylgruppe, X einen Rest der Formel $CH_2$, $R_2$ eine Phenethylgruppe, $R_5$ und $R_6$ ein Wasserstoffatom bedeuten.

11. Verbindung nach Anspruch 1, wobei R ein Wasserstoffatom, $R_1$ eine Ethylgruppe, X ein Schwefelatom, $R_2$ eine Phenethylgruppe und $R_5$ und $R_6$ ein Wasserstoffatom bedeuten.

12. Verbindung nach Anspruch 1, wobei R und $R_1$ ein Wasserstoffatom, X einen Rest der Formel $CH_2$, $R_5$ und $R_6$ ein Wasserstoffatom und $R_2$ eine Phenethylgruppe bedeuten.

13. Verbindung nach Anspruch 1, wobei R und $R_1$ eine Ethylgruppe, X einen Rest der Formel $CH_2$, $R_5$ und $R_6$ ein Wasserstoffatom und $R_2$ eine Phenethylgruppe bedeuten.

14. Verbindung nach Anspruch 1, wobei R und $R_1$ ein Wasserstoffatom, X ein Schwefelatom, $R_5$ und $R_6$ ein Wasserstoffatom und $R_2$ eine Phenethylgruppe bedeuten.

15. Verbindung nach Anspruch 1, wobei R und $R_1$ eine Ethylgruppe, X ein Schwefelatom, $R_5$ und $R_6$ ein Wasserstoffatom und $R_2$ eine Phenethylgruppe bedeuten.

16. Verfahren zur Herstellung von Verbindungen der Formel I nach Anspruch 1 und der pharmazeutisch verträglichen Salze davon, umfassend die Kopplung eines kondensierten Lactams der Formel

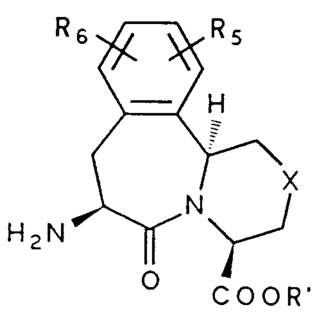

mit einem Reaktanten der Formeln

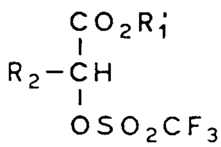

A

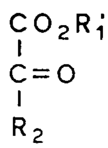

B

oder

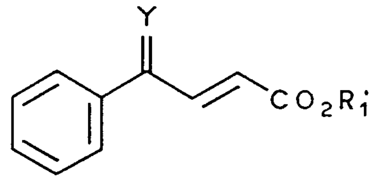

C

in denen R' und $R_1'$ , außer Wasserstoff, dieselben Bedeutungen wie R und $R_1$ in Anspruch 1 haben, $R_2$, $R_5$, $R_6$ und X dieselben Bedeutungen wie in Anspruch 1 haben, und Y Sauerstoff ist, und gegebenenfalls die Entfernung einer oder beider $R_1'$ - und R'-Esterfunktionen, sobald die Kopplung stattgefunden hat, wobei die Kopplung mit einer Verbindung der Formel B durch Zusammenbringen der Reaktanten in Gegenwart eines Molekularsiebes zur Bildung einer Schiffschen Base und durch Reduzieren der Schiffschen Base, bevorzugt mit Natriumcyanoborhydrid, durchgeführt wird, und die Kopplung mit einer Verbindung der Formel C gemäß einer 1,4-Michael-Additionsreaktion und anschließend Reduktion von Y unter Verwendung katalytischer Hydrierungsverfahren durchgeführt wird.

**17.** Verfahren zur Herstellung von Verbindungen der Formel

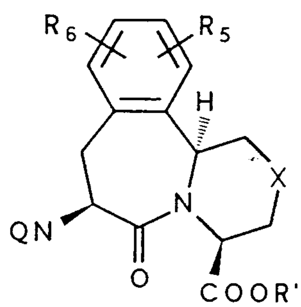

in der Q (H,H) oder eine N-Schutzgruppe bedeutet, R' wie in Anspruch 16 definiert ist, und X einen Rest der Formel $CH_2$, ein Schwefel- oder Sauerstoffatom bedeutet, umfassend eine Friedel-Crafts-Cyclisierung einer Verbindung der Formel

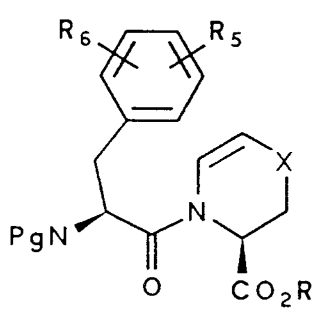

und gegebenenfalls die Entfernung jeder N-Schutzgruppe, wobei die Cyclisierung mit einer Lewis-Säure durchgeführt wird.

**18.** Verfahren nach Anspruch 17, wobei die Lewis-Säure eine Perfluoralkylsulfonsäure ist.

**19.** Verfahren nach Anspruch 17, wobei Q eine Phthalimidogruppe ist.

**20.** Verfahren nach Anspruch 17, wobei die Phthalimidoschutzgruppe durch Umsetzung mit Hydrazinhydrat entfernt wird.

**21.** Verbindung nach Anspruch 1 bis 15 zur Verwendung als Arzneimittel.

**22.** Verwendung einer Verbindung nach Anspruch 1 bis 15 zur Herstellung eines Arzneimittels zur Behandlung von Hypertonie.

**23.** Arzneimittel, umfassend eine Verbindung nach den Ansprüchen 1 bis 15 als Gemisch mit einem pharmazeutisch verträglichen Träger.

**24.** Arzneimittel, umfassend eine Verbindung nach den Ansprüchen 1 bis 15 zur gemeinsamen Verabreichung mit einem weiteren Antihypertonikum und/oder einem Diuretikum und/oder einem Blocker des Calciumrezeptors.

**25.** Arzneimittel, umfassend eine Verbindung nach den Ansprüchen 1 bis 15 und einen oder zwei weitere, aus einem anderen Antihypertonikum, einem Diuretikum und einem Blocker des Calciumrezeptors ausgewählte Wirkstoffe.

**Patentansprüche für folgende Vertragsstaaten : AT, ES, GR**

**1.** Verfahren zur Herstellung von Verbindungen der Formel

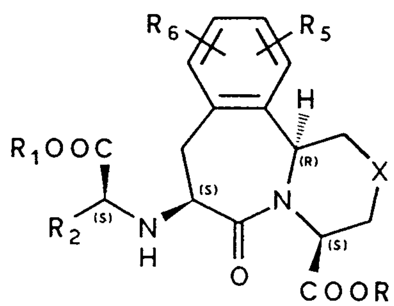

(I)

und die pharmazeutisch verträglichen Salze davon, wobei
R und $R_1$ unabhängig voneinander
(a) ein Wasserstoffatom;
(b) einen $C_1$-$C_6$-Alkylrest;
(c) einen substituierten $C_1$-$C_6$-Alkylrest, in dem die Substituenten Hydroxylgruppen, $C_1$-$C_4$-Alkyloxy- und Di-($C_1$-$C_4$)-alkylaminoreste sind;
(d) einen $C_6$-$C_{12}$-Arylrest;
(e) einen substituierten $C_6$-$C_{12}$-Arylrest, in dem die Substituenten $C_1$-$C_6$-Alkylreste; Halogenatome (F, Cl, Br, I) und $C_1$-$C_4$-Alkyloxyreste sind;
(f) einen Hetero-($C_4$-$C_9$)-arylrest, in dem das Heteroatom ein Sauerstoff-, Stickstoff- oder Schwefelatom sein kann;
(g) einen substituierten Hetero-($C_4$-$C_9$)-arylrest, in dem das Heteroatom ein Sauerstoff-, Stickstoff- oder Schwefelatom sein kann, und die Substituenten $C_1$-$C_6$-Alkylreste, Halogenatome (F, Cl, Br, I) und $C_1$-$C_4$-Alkyloxyreste sind,
bedeuten;
$R_2$
(a) ein Wasserstoffatom;
(b) einen unverzweigten oder verzweigten $C_1$-$C_8$-Alkylrest;
(c) einen unverzweigten oder verzweigten $C_2$-$C_8$-Alkenylrest;
(d) einen unverzweigten oder verzweigten $C_2$-$C_8$-Alkinylrest;
(e) einen $C_3$-$C_{10}$-Cycloalkylrest;
(f) einen $C_6$- oder $C_{10}$-Aryl-($C_1$-$C_4$)-alkylrest;
(g) einen substituierten $C_1$-$C_8$-Alkylrest, der einen Rest der Formel $CONR_2$, $SO_2NR_2$, $NRCONR_2$, $OCONR_2$, $NRCOOR$ oder $NR_2$ enthält, wobei R wie vorstehend definiert ist,

bedeutet;
$R_5$ und $R_6$ jeweils unabhängig voneinander
(a) ein Wasserstoffatom, eine Hydroxylgruppe;
(b) ein Halogenatom (F, Br, Cl, I);
(c) einen $C_1$-$C_6$-Alkylrest;
(d) einen $C_1$-$C_6$-Alkyloxyrest
bedeuten;
und X ein Rest der Formel $CH_2$, ein Sauerstoff- oder Schwefelotom ist,
umfassend die Kopplung eines kondensierten Lactams der Formel

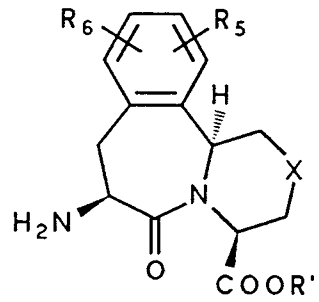

mit einem Reaktanten der Formeln

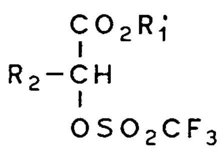

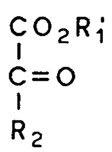

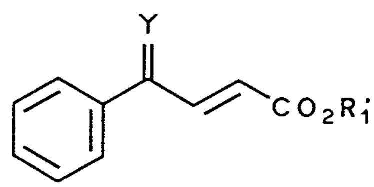

A B oder C

in denen R' und $R_1'$ , außer Wasserstoff, dieselben Bedeutungen wie R und $R_1$ in Anspruch 1 haben, $R_2$, $R_5$, $R_6$ und X vorstehende Bedeutungen haben, und Y Sauerstoff ist, und gegebenenfalls die Entfernung einer oder beider $R_1'$ - und R'-Esterfunktionen, sobald die Kopplung stattgefunden hat, wobei die Kopplung mit einer Verbindung der Formel B durch Zusammenbringen der Reaktanten in Gegenwart eines Molekularsiebes zur Bildung einer Schiffschen Base und durch Reduzieren der Schiffschen Base, bevorzugt mit Natriumcyanoborhydrid, durchgeführt wird, und die Kopplung mit einer Verbindung der Formel C gemäß einer 1,4-Michael-Additionsreaktion und anschließend Reduktion von Y unter Verwendung katalytischer Hydrierungsverfahren durchgeführt wird.

**2.** Verfahren zur Herstellung von Verbindungen der Formel

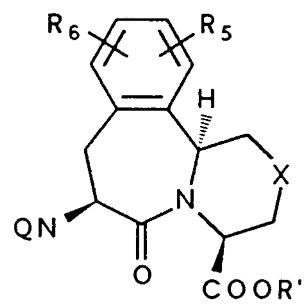

in der Q (H,H) oder eine N-Schutzgruppe bedeutet, R' wie in Anspruch 1 definiert ist, und X einen Rest

der Formel $CH_2$, ein Schwefel- oder Sauerstoffatom bedeutet, umfassend eine Friedel-Crafts-Cyclisierung einer Verbindung der Formel

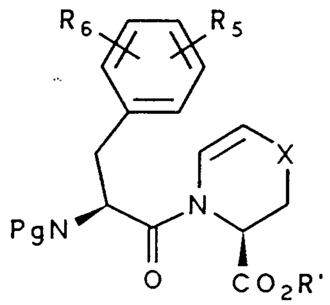

in der Pg eine N-Schutzgruppe bedeutet, und gegebenenfalls die Entfernung jeder N-Schutzgruppe, wobei die Cyclisierung mit einer Lewis-Säure durchgeführt wird.

**3.** Verfahren nach Anspruch 2, wobei die Lewis-Säure eine Perfluoralkylsulfonsäure ist.

**4.** Verfahren nach Anspruch 2, wobei Q eine Phthalimidogruppe ist.

**5.** Verfahren nach Anspruch 4, wobei die Phthalimidoschutzgruppe durch Umsetzung mit Hydrazinhydrat entfernt wird.

**6.** Verfahren gemäß den Ansprüchen 1 bis 5 zur Herstellung einer Verbindung, in der $R_5$ und $R_6$ ein Wasserstoffatom bedeuten.

**7.** Verfahren gemäß den Ansprüchen 1 bis 5 zur Herstellung einer Verbindung, in der $R_5$ eine Hydroxylgruppe und $R_6$ ein Wasserstoffatom bedeuten.

**8.** Verfahren gemäß den Ansprüchen 1 bis 5 zur Herstellung einer Verbindung, in der R ein Wasserstoffatom bedeutet.

**9.** Verfahren gemäß den Ansprüchen 1 bis 5 zur Herstellung einer Verbindung, in der $R_2$ eine Phenethylgruppe und R ein Wasserstoffatom bedeuten.

**10.** Verfahren gemäß den Ansprüchen 1 bis 5 zur Herstellung einer Verbindung, in der X einen Rest der Formel $CH_2$, R ein Wasserstoffatom und $R_2$ eine Phenethylgruppe bedeuten.

**11.** Verfahren gemäß den Ansprüchen 1 bis 5 zur Herstellung einer Verbindung, in der $R_1$ eine Ethylgruppe, R ein Wasserstoffatom, $R_2$ eine Phenethylgruppe und X einen Rest der Formel $CH_2$ bedeuten.

**12.** Verfahren gemäß den Ansprüchen 1 bis 5 zur Herstellung einer Verbindung, in der X ein Schwefelatom, R ein Wasserstoffatom, $R_1$ eine Ethylgruppe und $R_2$ eine Phenethylgruppe bedeuten.

**13.** Verfahren gemäß den Ansprüchen 1 bis 5 zur Herstellung einer Verbindung, in der X ein Sauerstoffatom, R ein Wasserstoffatom, $R_1$ eine Ethylgruppe und $R_2$ eine Phenethylgruppe bedeuten.

**14.** Verfahren gemäß den Ansprüchen 1 bis 5 zur Herstellung einer Verbindung, in der R ein Wasserstoffatom, $R_1$ eine Ethylgruppe, X einen Rest der Formel $CH_2$, $R_2$ eine Phenethylgruppe, $R_5$ und $R_6$ ein Wasserstoffatom bedeuten.

**15.** Verfahren gemäß den Ansprüchen 1 bis 5 zur Herstellung einer Verbindung, in der R ein Wasserstoffatom, $R_1$ eine Ethylgruppe, X ein Schwefelatom, $R_2$ eine Phenethylgruppe und $R_5$ und $R_6$ ein Wasserstoffatom bedeuten.

**16.** Verfahren gemäß den Ansprüchen 1 bis 5 zur Herstellung einer Verbindung, in der R und $R_1$ ein Wasserstoffatom, X einen Rest der Formel $CH_2$, $R_5$ und $R_6$ ein Wasserstoffatom und $R_2$ eine

Phenethylgruppe bedeuten.

**17.** Verfahren gemäß den Ansprüchen 1 bis 5 zur Herstellung einer Verbindung, in der R und $R_1$ eine Ethylgruppe, X einen Rest der Formel $CH_2$, $R_5$ und $R_6$ ein Wasserstoffatom und $R_2$ eine Phenethylgruppe bedeuten.

**18.** Verfahren gemäß den Ansprüchen 1 bis 5 zur Herstellung einer Verbindung, in der R und $R_1$ ein Wasserstoffatom, X ein Schwefelatom, $R_5$ und $R_6$ ein Wasserstoffatom und $R_2$ eine Phenethylgruppe bedeuten.

**19.** Verfahren gemäß den Ansprüchen 1 bis 5 zur Herstellung einer Verbindung, in der R und $R_1$ eine Ethylgruppe, X ein Schwefelatom, $R_5$ und $R_6$ ein Wasserstoffatom und $R_2$ eine Phenethylgruppe bedeuten.

**20.** Verwendung einer Verbindung nach einem der vorstehenden Ansprüche zur Herstellung eines Arzneimittels zur Behandlung von Hypertonie.

**Revendications**
**Revendications pour les Etats contractants suivants : BE, CH, DE, FR, GB, IT, LU, NL, SE**

**1.** Composés de formule :

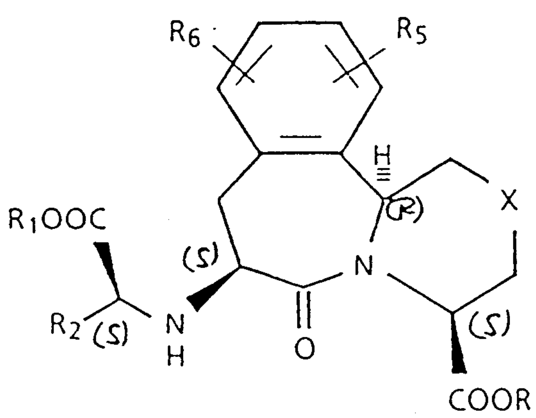

(I)

et leurs sels pharmaceutiquement acceptables, formule dans laquelle :
R et $R_1$ représentent indépendamment :
(a) l'hydrogène ;
(b) un groupe $C_1$-$C_6$ alkyle ;
(c) un groupe $C_1$-$C_6$ alkyle substitué dont les substituants sont les suivants : hydroxy, $C_1$-$C_4$ alkyloxy et di-($C_1$-$C_4$)-alkylamino ;
(d) un groupe $C_6$-$C_{12}$ aryle ;
(e) un groupe $C_6$-$C_{12}$ aryle substitué dont les substituants sont les suivants : $C_1$-$C_6$ alkyle, halo (F, Cl, Br, I) et $C_1$-$C_4$ alkyloxy ;
(f) un groupe hétéro ($C_4$-$C_9$) aryle dont l'hétéroatome peut être l'un des suivants : O, N ou S ;
(g) un groupe hétéro ($C_4$-$C_9$) aryle substitué dont l'hétéroatome peut être l'un des suivants : O, N ou S et dont les substituants sont les suivants : $C_1$-$C_6$ alkyle, halo (F, Cl, Br, I) et $C_1$-$C_4$ alkyloxy ;
$R_2$ est
(a) l'hydrogène ;
(b) un groupe $C_1$-$C_8$ alkyle linéaire ou ramifié ;
(c) un groupe $C_2$-$C_8$ alcényle linéaire ou ramifié ;
(d) un groupe $C_2$-$C_8$ alcynyle linéaire ou ramifié ;
(e) un groupe $C_3$-$C_{10}$ cycloalkyle ;

(f) un groupe $C_6$ ou $C_{10}$ aryl ($C_1$-$C_4$) alkyle ;
(g) un groupe $C_1$-$C_8$ alkyle substitué qui contient un groupe $CONR_2$, $SO_2NR_2$, $NRCONR_2$, $OCONR_2$, NRCOOR ou $NR_2$, où R est comme défini précédemment ;

$R_5$ et $R_6$ désignent chacun indépendamment :
(a) l'hydrogène, un groupe hydroxy ;
(b) un groupe halo (F, Br, Cl, I) ;
(c) un groupe $C_1$-$C_6$ alkyle ;
(d) un groupe $C_1$-$C_6$ alkyloxy ; et X est $CH_2$, O ou S.

**2.** Un composé selon la revendication 1, selon lequel $R_5$ et $R_6$ sont de l'hydrogène.

**3.** Un composé selon la revendication 1, selon lequel $R_5$ est un groupe hydroxy et $R_6$ est l'hydrogène.

**4.** Un composé selon la revendication 1, selon lequel R est H.

**5.** Un composé selon la revendication 4, selon lequel $R_2$ est un groupe phénéthyle.

**6.** Un composé selon la revendication 5, selon lequel X est $CH_2$.

**7.** Un composé selon la revendication 6, selon lequel $R_1$ est un groupe éthyle.

**8.** Un composé selon la revendication 7, selon lequel X est S.

**9.** Un composé selon la revendication 7, selon lequel X est O.

**10.** Un composé selon la revendication 1, selon lequel R est H, $R_1$ est éthyle, X est $CH_2$, $R_2$ est phénéthyle, $R_5$ et $R_6$ sont H.

**11.** Un composé selon la revendication 1, selon lequel R est H, $R_1$ est éthyle, X est S, $R_2$ est phénétyle, et $R_5$ et $R_6$ sont H.

**12.** Un composé selon la revendication 1, selon lequel R et $R_1$ sont H, X est $CH_2$, $R_5$ et $R_6$ sont H et $R_2$ est phénéthyle.

**13.** Un composé selon la revendication 1, selon lequel R et $R_1$ sont éthyle, X est $CH_2$, $R_5$ et $R_6$ sont H et $R_2$ est phénéthyle.

**14.** Un composé selon la revendication 1, selon lequel R et $R_1$ sont H, X est S, $R_5$ et $R_6$ sont H et $R_2$ est phénéthyle.

**15.** Un composé selon la revendication 1, selon lequel R et $R_1$ sont éthyle, X est S, $R_5$ et $R_6$ sont H et $R_2$ est phénéthyle.

**16.** Un procédé de préparation du composé de formule I selon la revendication 1 et de leurs sels pharmaceutiquement acceptables qui comprend le couplage d'un lactame condensé de formule :

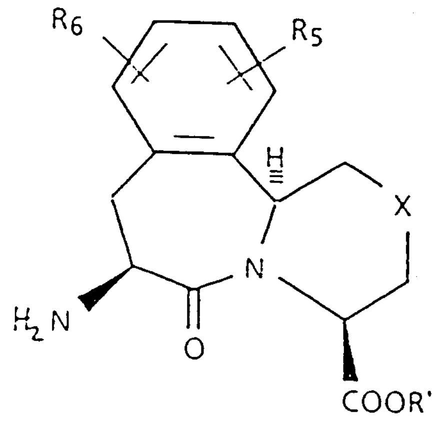

avec un réactif de formules

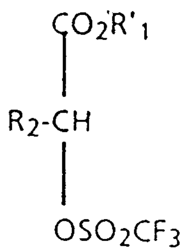

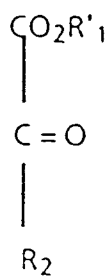

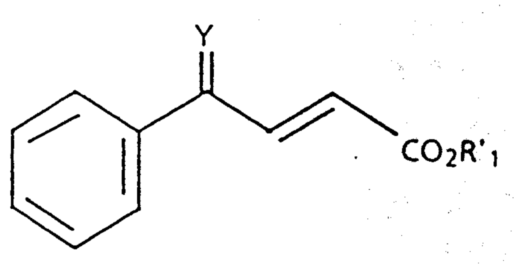

A B C

ou

dans lesquelles R' et $R'_1$ ont les mêmes significations que R et $R_1$ dans la revendication 1 à l'exception de l'hydrogène, $R_2$, $R_5$, $R_6$ et X ont les mêmes significations que dans la revendication 1, et Y est oxygén et éventuellement l'élimination de l'un ou des deux fonctions ester $R'_1$ et R' après ledit couplage, ce couplage avec un composé de formule B étant effectué par mise en contact des reactifs ensemble en présence d'un tamis moléculaire pour former une base de Schiff et la réduction de cette base de Schiff de préférence avec le cyanoborohydrure de sodium, ledit couplage avec un composé de formule C étant effectué conformément à la réaction d'addition de 1,4-Michael, suivie de la réduction de Y en utilisant des techniques d'hydrogénation catalytique.

**17.** Un procédé de préparation des composés de formule :

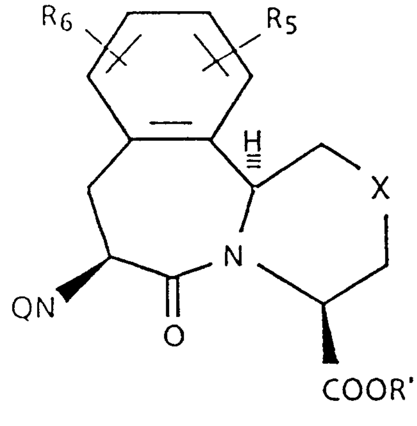

dans laquelle Q est (H,H) ou un groupe N-protecteur, R' est comme défini dans la revendication 16 et X

est $CH_2$, S, ou O qui consiste à soumettre un composé de formule :

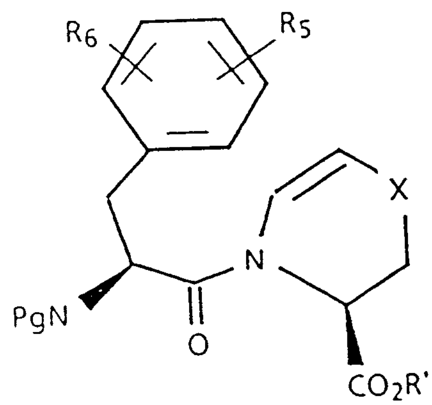

à une cyclisation de Friedel-Crafts, et éventuellement à éliminer tout groupe N-protecteur, cette cyclisation étant effectuée à l'aide d'un acide de Lewis.

**18.** Un procédé selon la revendication 17, selon lequel l'acide de Lewis est un acide perfluoroalkylsulfonique.

**19.** Un procédé selon la revendication 17, selon lequel Q est un groupe phtalimido.

**20.** Un procédé selon la revendication 17, selon lequel le groupe protecteur phtalimido est éliminé par réaction avec de l'hydrate d'hydrazine.

**21.** Un composé selon l'une des revendications 1 à 15 à utiliser comme médicament.

**22.** Utilisation d'un composé selon l'une des revendications 1 à 15 pour la préparation d'un médicament pour le traitement de l'hypertension.

**23.** Une composition pharmaceutique qui comprend un composé selon les revendications 1 à 15 en mélange avec un support pharmaceutiquement acceptable.

**24.** Un médicament qui comprend un composé selon les revendications 1 à 15 pour l'administration conjointe avec un autre agent antihypertenseur et/ou diurétique et/ou un inhibiteur calcique.

**25.** Une composition pharmaceutique qui comprend un composé selon les revendications 1 à 15 et un ou deux autres ingrédients actifs choisis parmi un autre antihypertenseur, un diurétique et un inhibiteur calcique.

**Revendications pour les Etats contractants suivants : AT, ES, GR**

**1.** Un procédé de préparation du composé de formule :

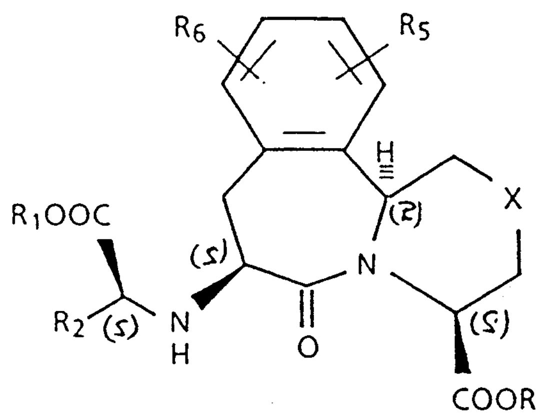

(I)

et de leurs sels pharmaceutiquement acceptables, formule dans laquelle :

R et $R_1$ représentent indépendamment :

(a) l'hydrogène ;

(b) un groupe $C_1$-$C_6$ alkyle ;

(c) un groupe $C_1$-$C_6$ alkyle substitué dont les substituants sont les suivants : hydroxy, $C_1$-$C_4$ alkyloxy et di-($C_1$-$C_4$)-alkylamino ;

(d) un groupe $C_6$-$C_{12}$ aryle ;

(e) un groupe $C_6$-$C_{12}$ aryle substitué dont les substituants sont les suivants : $C_1$-$C_6$ alkyle, halo (F, Cl, Br, I) et $C_1$-$C_4$ alkyloxy ;

(f) un groupe hétéro ($C_4$-$C_9$) aryle dont l'hétéroatome peut être l'un des suivants : O, N ou S ;

(g) un groupe hétéro ($C_4$-$C_9$) aryle substitué dont l'hétéroatome peut être l'un des suivants : O, N ou S et dont les substituants sont les suivants : $C_1$-$C_6$ alkyle, halo (F, Cl, Br, I) et $C_1$-$C_4$ alkyloxy ;

$R_2$ est

(a) l'hydrogène ;

(b) un groupe $C_1$-$C_8$ alkyle linéaire ou ramifié ;

(c) un groupe $C_2$-$C_8$ alcényle linéaire ou ramifié ;

(d) un groupe $C_2$-$C_8$ alcynyle linéaire ou ramifié ;

(e) un groupe $C_3$-$C_{10}$ cycloalkyle ;

(f) un groupe $C_6$ ou $C_{10}$ aryl ($C_1$-$C_4$) alkyle ;

(g) un groupe $C_1$-$C_8$ alkyle substitué qui contient un groupe $CONR_2$, $SO_2NR_2$, $NRCONR_2$, $OCONR_2$, $NRCOOR$ ou $NR_2$, où R est comme défini précédemment ;

$R_5$ et $R_6$ désignent chacun indépendamment :

(a) l'hydrogène, un groupe hydroxy ;

(b) un halo (F, Br, Cl, I) ;

(c) un groupe $C_1$-$C_6$ alkyle ;

(d) un groupe $C_1$-$C_6$ alkyloxy ; et X est $CH_2$, O ou S, qui comprend le couplage d'un lactame condensé de formule :

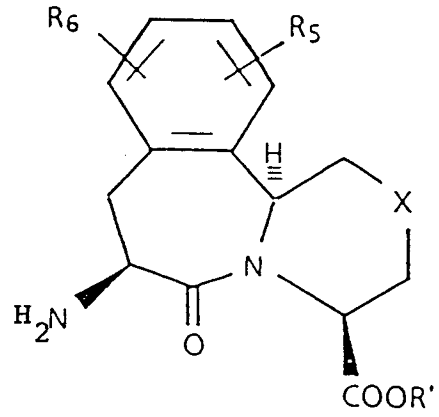

avec un réactif de formules

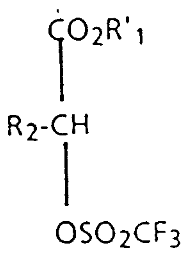 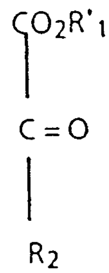 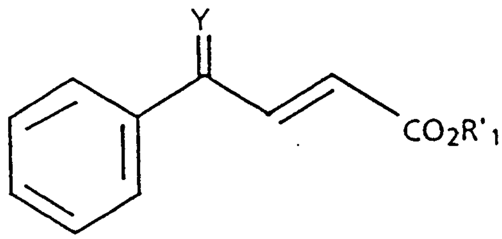

A B ou C

dans lesquelles R' et $R'_1$ on les mêmes significations que R et $R_1$ dans la revendication 1 à l'exception de l'hydrogène, $R_2$, $R_5$, $R_6$ et X ont les mêmes significations ci dessus, et Y oxygén et éventuellement l'élimination de l'une ou des deux fonctions ester $R'_1$ et R' après ledit couplage, ce couplage avec un composé de formule B étant effectué par mise en contact des réactifs ensemble en présence d'un tamis moléculaire pour former un base de Schiff et la réduction de cette base de Schiff est de préférence avec le cyanoborohydrure de sodium, ledit couplage avec un composé de formule C étant effectué conformément à la réaction d'addition de 1,4-Michael, suivie par la réduction de Y en utilisant les techniques d'hydrogénation catalytique.

**2.** Un procédé de préparation du composé de formule :

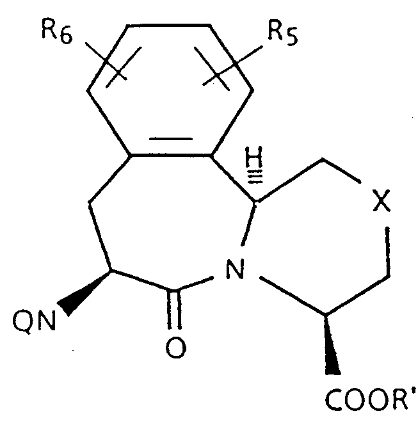

dans laquelle Q est (H,H) ou un groupe N-protecteur, R' est comme défini dans la revendication 1 et X est $CH_2$, S, ou O qui consiste à soumettre un composé de formule :

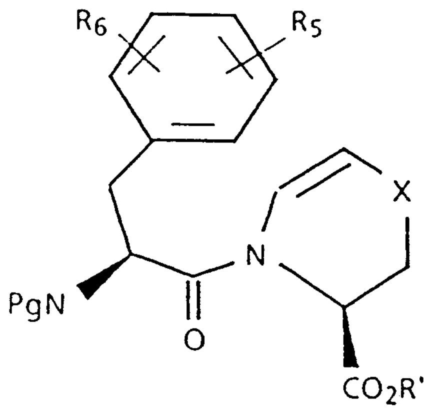

dans laquelle $P_g$ est un groupe N-protecteur, à une cyclisation de Friedel-Crafts et éventuellement l'élimination de tout groupe N-protecteur, cette cyclisation étant effectuée par un acide de Lewis.

3. Un procédé selon la revendication 2, selon lequel l'acide de Lewis est un acide perfluoroalkylsulfonique.

4. Un procédé selon la revendication 2, selon lequel Q est un groupe phtalimido.

5. Un procédé selon la revendication 4, selon lequel le groupe protecteur phtalimido est éliminé par réaction avec l'hydrate d'hydrazine.

6. Un procédé selon les revendications 1 à 5, pour la préparation d'un composé dans lequel $R_5$ et $R_6$ sont de l'hydrogène.

7. Un procédé selon les revendications 1 à 5 pour la préparation d'un composé selon lequel $R_5$ est un groupe hydroxy et $R_6$ est l'hydrogène.

8. Un procédé selon les revendications 1 à 5, pour la préparation d'un composé dans lequel R est H.

9. Un procédé selon les revendications 1 à 5, pour la préparation d'un composé dans lequel $R_2$ est un groupe phénéthyle et R est l'hydrogène.

10. Un procédé selon les revendications 1 à 5, pour la préparation d'un composé dans lequel X est $CH_2$, R est H et $R_2$ est phénéthyle.

11. Un procédé selon les revendications 1 à 5, pour la préparation d'un composé dans lequel $R_1$ est éthyle, R est H, $R_2$ est phénéthyle et X est $CH_2$.

12. Un procédé selon les revendications 1 à 5, pour la préparation d'un composé dans lequel X est S, R est H, $R_1$ est éthyle et $R_2$ est phénéthyle.

13. Un procédé selon les revendications 1 à 5, pour la préparation d'un composé dans lequel X est O, R est n, $R_1$ est éthyle et $R_2$ phénéthyle.

14. Un procédé selon les revendications 1 à 5, pour la préparation d'un composé dans lequel R est H, $R_1$ est éthyle, X est $CH_2$, $R_2$ est phénéthyle, $R_5$ et $R_6$ sont H.

15. Un procédé selon les revendications 1 à 5, pour la préparation d'un composé dans lequel R est H, $R_1$ est éthyle, X est S, $R_2$ est phénéthyle et $R_5$ et $R_6$ sont H.

16. Un procédé selon les revendications 1 à 5, pour la préparation d'un composé dans lequel R et $R_1$ sont H, X est $CH_2$, $R_5$ et $R_6$ sont H et $R_2$ est phénéthyle.

**17.** Un procédé selon les revendications 1 à 5, pour la préparation d'un composé dans lequel R et $R_1$ sont éthyle, X est $CH_2$, $R_5$ et $R_6$ sont H et $R_2$ est phénéthyle.

**18.** Un procédé selon les revendications 1 à 5, pour la préparation d'un composé dans lequel R et $R_1$ sont H, X est S, $R_5$ et $R_6$ sont H et $R_2$ est phénéthyle.

**19.** Un procédé selon les revendications 1 à 5, pour la préparation d'un composé dans lequel R et $R_1$ sont éthyle, X est S, $R_5$ et $R_6$ sont H et $R_2$ est phénéthyle.

**20.** Utilisation d'un composé de l'une quelconque des revendications précédentes pour la préparation d'un médicament pour le traitement de l'hypertension.